# EUROPEAN PATENT SPECIFICATION

(11) **EP 1 542 528 B1**
(45) Date of publication and mention of the grant of the patent: **23.09.2009**
(21) Application number: 03792044.4
(22) Date of filing: 22.08.2003
(51) Int. Cl.: C12N 15/09, C12N 15/63, A61K 38/19, A61K 48/00, A61K 39/395, A61P 29/00

(54) **A METHOD OF TREATMENT AND PROPHYLAXIS**
BEHANDLUNGS- UND PROPHYLAXEVERFAHREN
METHODE DE TRAITEMENT ET DE PROPHYLAXIE

(30) Priority: 23.08.2002 AU 2002950957; 29.08.2002 AU 2002951071
(43) Date of publication of application: 22.06.2005
(73) Proprietor: THE WALTER AND ELIZA HALL INSTITUTE OF MEDICAL RESEARCH, Parkville, VIC 3052 (AU)
(72) Inventor: LAWLOR, Kathryn, Elizabeth, Kensington, Victoria 3031 (AU); WICKS, Ian, Peter, Kew, Victoria 3101 (AU); CAMPBELL, Ian, Keith, Wantirna South, Victoria 3152 (AU); ROBERTS, Andrew, Warwick, Camberwell, Victoria 3124 (AU); METCALF, Donald, Balwyn, Victoria 3103 (AU)
(74) Representative: Dzieglewska, Hanna Eva
(86) International application number: PCT/AU2003/001078
(87) International publication number: WO 2004/017727

(56) References cited:
- WO-A-02/07676
- WO-A-95/21864
- WO-A-95/21867
- US-A- 5 420 109
- US-A- 5 639 455
- US-A1- 2002 141 994
- CAPORALI ROBERTO ET AL: "Cyclophosphamide plus granulocyte colony stimulating factor (G-CSF) is more effective than G-CSF alone in mobilizing hemopoietic progenitor cells in severe, refractory rheumatoid arthritis" HAEMATOLOGICA, vol. 86, no. 1, January 2001 (2001-01), pages 106-107, XP002338389 ISSN: 0390-6078
- GHIVIZZANI STEVEN C. ET AL.: 'Gene therapy approaches for treating rheumatoid arthritis' CLINICAL ORTHOPAEDICS AND RELATED RESEARCH no. 379 SUPPL., October 2000, pages S288 - S299, XP009028678 Ref: 50
- SAGGIO I. ET AL.: 'Adenovirus-mediated gene transfer of a human IL-6 antagonist' GENE THERAPY vol. 4, no. 8, August 1997, pages 839 - 845, XP002054218
- CAMPBELL IAN K. ET AL.: 'The colony-stimulating factors and collagen-induced arthritis: exacerbation of disease by M-CSF and G-CSF and requirement for endogenous M-CSF' JOURNAL OF LEUKOCYTE BIOLOGY vol. 68, no. 1, July 2000, pages 144 - 150, XP008051320
- ROESSLER BLAKE J. ET AL.: 'Inhibition of interleukin-1-induced effects in synoviocytes transduced with the human IL-1 receptor antagonist cDNA using an adenoviral vector' HUMAN GENE THERAPY vol. 6, no. 3, March 1995, pages 307 - 316, XP008051108
- BERNARD L.A. ET AL.: 'Pimecrolimus 1% cream (Elidel) for atopic dermatitis' SKIN THERAPY LETTER vol. 7, no. 4, April 2002, pages 1 - 3, XP008051109
- KAWAMURA H. ET AL: 'Expansion of extrathymic T cells as well as granulocytes in the liver and other organs of granulocyte-colony stimulating factor transgenic mice: why they lost the ability of hybrid resistance' THE JOURNAL OF IMMUNOLOGY vol. 162, no. 10, 15 May 1999, pages 5957 - 5964, XP002989410
- HERMANS M.H. ET AL: 'Sustained receptor activation and hyperproliferation in response to granulocyte colony-stimulating factor (G-CSF) in mice with a severe congenital neutropenia/acute myeloid leukemia-derived mutation in the G-CSF receptor gene' JOURNAL OF EXPERIMENTAL MEDICINE vol. 189, no. 4, 15 February 1999, pages 683 - 692, XP002988857

## Description

### FIELD OF THE INVENTION

The present invention relates generally to an agent for the treatment or prophylaxis of arthritis or to the use of an agent in the manufacture of a medicament for such a purpose.
The present invention further provides an in-vivo non-therapeutic method using a non-humain animal model for screening for agents useful in the treatment or prophylaxis of arthritis.

### BACKGROUND OF THE INVENTION

Bibliographic details of references provided in the subject specification are listed at the end of the specification.

Reference to any prior art in this specification is not, and should not be taken as, an acknowledgement or any form of suggestion that this prior art forms part of the common general knowledge in any country.

Granulocyte colony-stimulating factor (G-CSF, encoded by the *CSF-3* gene) is a hematopoietic growth factor that regulates the production of granulocytes (Nicola et al., Nature 314: 625, 1985; Metcalf International Journal of Cancer 25: 225, 1980; Nicola et al., Journal of Biological Chemistry 258: 9017, 1983). G-CSF mediates its effects through interaction with the granulocyte-colony stimulating factor receptor (G-CSFR, encoded by the *CSFR-3* gene), a member of the type I cytokines receptor superfamily (Demetri et al., Blood 78: 2791-2808, 1991). Major biological actions of G-CSF in humans and mice, include increasing the production and release of neutrophils from the bone marrow (Souza et al., Science 232: 61, 1986; Lord et al., Proc. Natl. Acad Sci. USA 86:9499-9503, 1989), mobilizing hematopoietic progenitor cells from the marrow into the peripheral blood *(*Bungart et al., British Journal of Haematology 22: 1156, 1990; de Haan et al., Blood 86: 2986-2992, 1995; Roberts et al., Blood 89: 2736-2744, 1997), and modulating the differentiation and effector functions of mature neutrophils (Yong et al., European Journal of Haematology 49: 251-259, 1992; Colotta et al., Blood 80: 2012-2020, 1992; Rex et al., Transfusion 35: 605-611, 1995; Gericke et al., Journal of Leukocyte Biology 57: 455-461, 1995; Xu et al., British Journal of Haematology 93: 558-568, 1996; Yong, British Journal of Haematology 94: 40-47, 1996; Jacob et al., Blood 92: 353-361, 1998). G-CSF is used to treat neutropenia, as well as mobilisation of haemopoietic stem cells (HSC) for autologous and allogenic stem cell transplantation (Welte et al., Blood 88: 1907-1929, 1996).

Use of G-CSF for HSC mobilization can cause exacerbations of rheumatoid arthritis (RA) *(*Snowden et al., Bone Marrow Transplantation 22: 1035-1041, 1998). G-CSF along with colony stimulating factors, GM-CSF and M-CSF are produced by human synovial fibroblasts and chondrocytes in response to IL-1 and TNF *in vitro* (Leizer et al., Blood 76: 1989-1996, 1990; Hamilton et al., Blood 79: 1413-1419, 1992), and G-CSF has been found in the serum and synovial fluid of RA patients *(*Tanabe et al., Rheumatology International 16: 67-76, 1996; Nakamura et al., Clinical and Experimental Rheumatology 18: 713-718, 2000). Systemic administration of G-CSF has been shown to exacerbate murine collagen-induced arthritis (CIA) with increased severity and incidence of disease in DBA/1 mice (Campbell et al., Journal of Leukocyte Biology 68: 144-150, 2000), as well as a passive transfer model of CIA in rats (Miyahara et al., Clinical Immunology and Immunopathology 69: 69-76, 1993). G-CSF transgenic mice have increased bone resorption and reduced bone formation (Takahashi et al., Laboratory Investigation 74: 827-834, 1996), indicating that G-CSF may have a role in bone turnover.

G-CSF is able to expand a monocyte/macrophage subset and induce anti-inflammatory cytokines that can protect against endotoxemia in mice *(*Gorgen et al., Journal of Immunology 149: 918, 1992). G-CSF has also been reported to impair allogeneic and mitogenic T cell responses in both humans and mice (Foster et al., Transplantation 59: 1557, 1995; Pan et al., Blood 86: 4422, 1995), and to cause a shift of the T cell cytokine profile towards Th2 cytokine production, with a corresponding reduction in Th1 IFN-γ expression (Pan *et al.,* 1995, *supra;* Franzke et al., Blood 102: 734-739). In murine studies, this deviation to Th2 cytokine production has been associated with protection against acute graft-versus-host disease, experimental autoimmune encephalomyelitis (EAE) and spontaneous systemic lupus erythematosus (Pan *et al.,* 1995, *supra;* Zavala et al., Journal of Immunology 163: 5125-5132, 1999; Zavala et al., Journal of Immunology 168: 2011-2019, 2000). Mice deficient in G-CSF were protected from neutrophil-mediated glomerulonephritis, but not T cell/macrophage-mediated glomerulonephritis *(*Kitching et al., Journal of the American Society of Nephrologh 13: 350-358, 2000).

G-CSF is, therefore, a pleiotropic molecule with a range of functions. There is a need to more fully characterize these functions and to elucidate if modulation of these functions can lead to health benefits.

### SUMMARY OF THE INVENTION

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

The role of neutrophils was studied in a murine model of arthritis. The murine model included use of antibodies to deplete neutrophils as well as the use of a G-CSF knockout mouse. It was determined that these mice were highly resistant to the induction of acute arthritis, but this effect did not seem to be explained by the lower neutrophil counts. It was similarly found that G-CSF could directly induce joint inflammation by local administration and that systemic G-CSF could substitute for IL-1 in the model of acute arthritis.

Collagen induced arthritis (CIA) is a chronic autoimmune model widely used to investigate rheumatoid arthritis (RA) pathogenesis and for evaluation of prospective therapies. To examine the requirement for endogenous G-CSF in chronic joint disease, G-CSF^{-/-} and wild-type (WT) mice were immunized with chick Type II collagen (CII) in Complete Freund's Adjuvant (CFA) to induce CIA. There was marked protection from disease in mice deficient in G-CSF, identifying a major role for G-CSF in CIA. T cell responses to CII were normal in G-CSF knockout mice.

Collectively, this shows that endogenous G-CSF plays a major role in inflammatory arthritis. Down-regulating G-CSF activity, locally or systemically or reducing levels of G-CSF or inhibiting or down-regulating the G-CSF receptor (G-CSFR), is proposed to be a useful mechanism to treat or reduce the severity of inflammatory conditions such as chronic inflammatory arthritis and rheumatoid arthritis or other chronic immune-mediated inflammatory disease conditions.

Accordingly, the present invention contemplates a use of an agent which inhibits the activity of G-CSF or G-CSFR and/or which reduces the level of expression of a gene encoding said G-CSF or G-CSFR in the manufacture of a medicament for the prophylaxis and/or treatment of arthritis in a subject.

The therapeutic efficacy of administration of a neutralising monoclonal antibody (mAb) to G-CSF was tested in the acute arthritis model. Inhibition of G-CSF during acute arthritis resulted in a dose dependent reduction in myeloid lineage cells in the BM, neutropenia in the peripheral blood and reduced cellular infiltration of the involved joint.

The present invention further provides an agent which inhibits the activity of G-CSF or G-CSFR and/or which reduces the level of expression of a gene encoding G-CSF or G-CSFR for treatment or prophylaxis of arthritis in a subject.

The present invention further provides an *in vivo* non-therapeutic method using a non-human animal model for identifying agents capable of inhibiting the activity of G-CSF and/or inhibiting the interaction of G-CSF with G-CSFR. A list of abbreviations used herein is provided in Table 1.

**TABLE 1**

| ***Abbreviations*** | |
|---|---|
| **ABREVIATION** | **DESCRIPTION** |
| acute arthritis | methylated bovine serum albumin/IL-1-induced arthritis |
| Ab | antibody |
| B6 | C57BL/6 |
| BM | bone marrow |
| BSA | bovine serum albumin |
| CFA | Complete Freund's Adjuvant |
| CIA | Collagen-induced arthritis |
| CII | Type II collagen |
| *CSF3*/ *Csf3* | Colony stimulating factor 3 gene (human/mouse) |
| *CSF3R* | Colony stimulating factor 3 receptor gene (human) |
| DNP | [dinitrophenyl] acetyl |
| EAE | Experimental autoimmune encephalomyelitis |
| FACS | fluorescence activated cell sorting |
| FCS | foetal calf serum |
| FITC | fluorescein isothiocyanate |
| G-CSF | granulocyte colony-stimulating factor |
| G-CSF^{-/-} | granulocyte colony-stimulating factor-deficient |
| G-CSFR | granulocyte colony-stimulating factor receptor |
| GM-CSF | granulocyte-macrophage colony stimulating factor |
| H&E | hemotoxylin and eosin |
| HRP | horse radish peroxidase |
| HSC | haemopoietic stem cell |
| i.a. | intra-articular (ly) |
| i.d. | intra-dermal (1y) |
| Ig | immunoglobulin |
| IL- | interleukin- |
| IFN-γ | interferon-gamma |
| i.p. | intra-peritoneal (1y) |
| i.v. | intra-venous |
| KLH | keyhole limpet cyanin |
| LN | lymph node |
| mAb | monoclonal antibody |
| mBSA | methylated bovine serum albumin |
| mBSA/IL-1-induced arthritis | methylated bovine serum albumin-induced arthritis |
| M-CSF | macrophage colony stimulating factor |
| M-CSFR | macrophage colony stimulating factor receptor |
| NMS | normal mouse serum |
| NP- | [4-hydroxy-3-nitrophenyl] acetyl |
| PBS | phosphate buffered saline |
| PE | phycoerythrin |
| RA | Rheumatoid arthritis |
| rHuG-CSF (filgrastim) | recombinant human G-CSF |
| s.c. | subcutaneous (ly) |
| TdR | thymidine |
| TNF | tumour necrosis factor |
| TNP | trinitrophenyl |
| WEHI | The Walter and Eliza Hall Institute of Medical Research |
| WT | wild type |

### BRIEF DESCRIPTION OF THE FIGURES

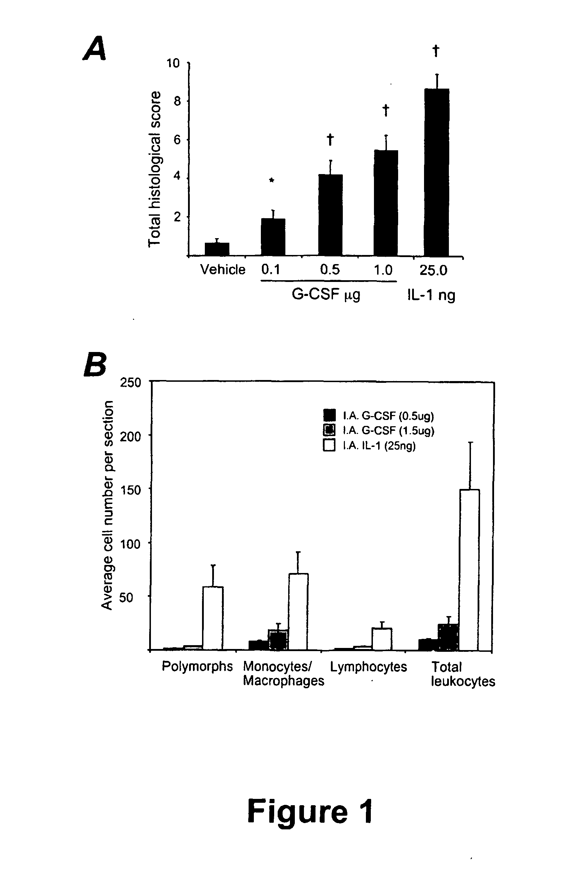

**Figure 1** is a graphical representation showing intra-articular effects of G-CSF. ***A*.** Mice were treated intra-articularly with *filgrastim* (G-CSF; 0.1, 0.5, or 1 µg), IL-1 (25 ng) or saline (vehicle) for three consecutive days and examined histologically at day 3. * P < 0.05; † P < 0.005 compared to saline control. ***B*.** Joint exudate cells in G-CSF (0.5, 1.5 µg) or IL-1 injected joints were quantified. There was a prominent monocyte/macrophage exudate in the G-CSF-injected joints. n > 12 joints per group.

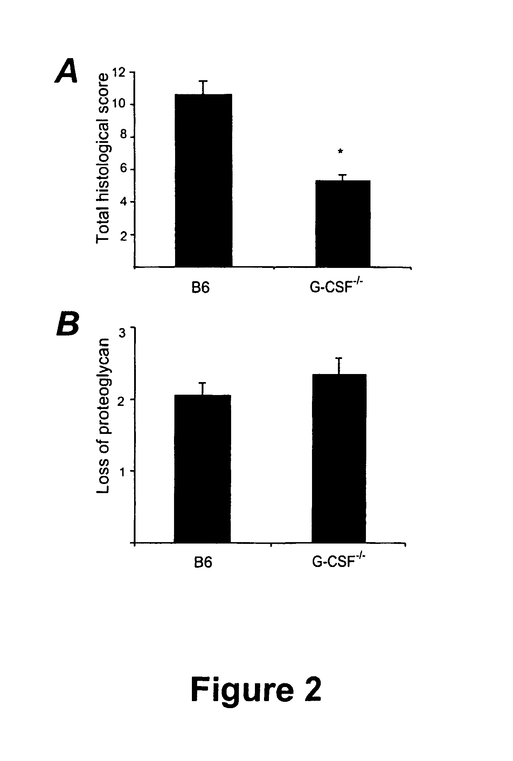

**Figures 2A and B** are graphical representations showing that G-CSF is necessary for local IL-1 induced joint inflammation, but not IL-1 induced proteoglycan loss. B6 and *G-CSF-l-*mice were injected i.a. with IL-1 (25 ng) on days 0, 1 and 2, and assessed histologically at day 3 for ***A*.** the severity of inflammatory and destructive features and ***B*.** loss of articular cartilage proteoglycan. Results are representative of the mean ± SEM (scored out of 5). Data are from 1 of 2 experiments; n = 14 joints/group; * p < 0.05.

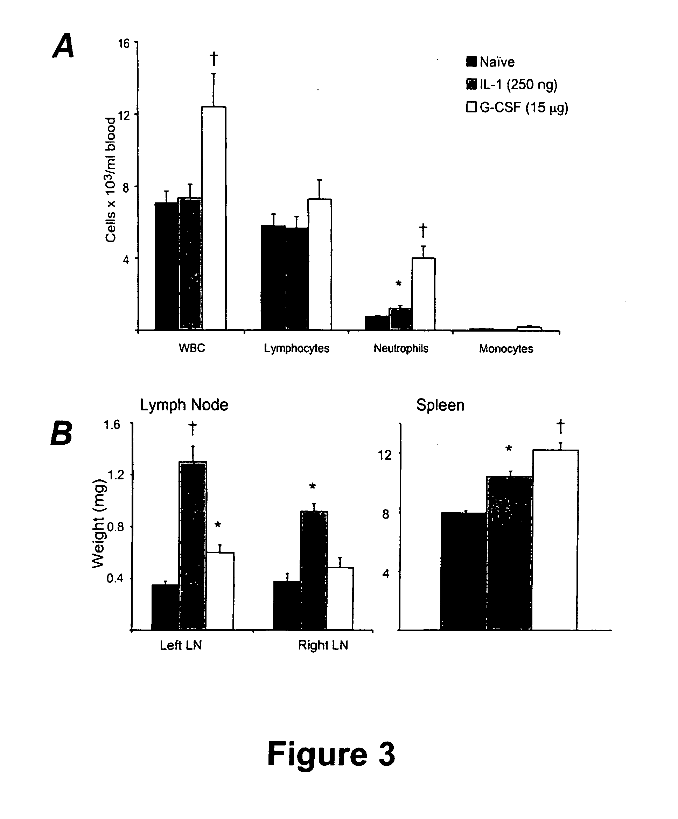

**Figure 3A and 3B** show graphical representations of the systemic effects of *filgrastim in lieu* of IL-1 in the acute mBSA/IL-1-induced model. B6 mice were injected i.a. with mBSA and s.c. on days 0, 1, 2 with saline [black bars], IL-1 (250 ng) [grey bars] or *filgrastim* (15 µg) [white bars]. Shown are ***A*.** Peripheral blood counts at day 2 and ***B*.** popliteal lymph node (LN) and spleen weights at day 7. Data are representative of 3 experiments with n > 5 mice per group. * P < 0.05; † P < 0.01 compared to mBSA/saline control group.

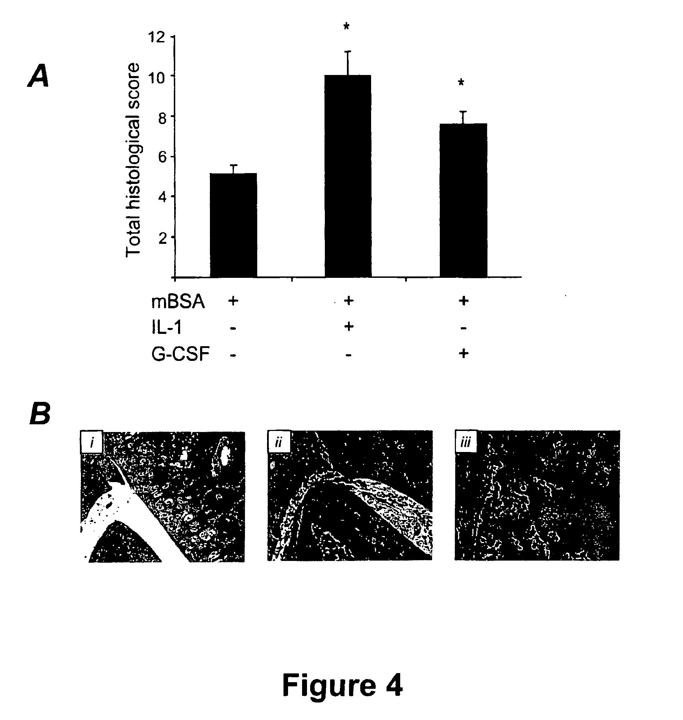

**Figure 4A and 4B** illustrate the effects of systemic G-CSF *in lieu* of IL-1 in the acute arthritis model. Mice were injected i.a. with mBSA and s.c. with either saline vehicle, IL-1, or G-CSF and the injected knees were examined histologically at day 7. ***A*.** Mean total histological scores ± SEM. ***B*** Representative histology illustrating ***i*** mBSA/saline treated joint with minimal exudate cells in the joint space (100X), ***ii*** mBSA/IL-1-treated joint with moderate to marked exudate and synovitis and ***iii*** mBSA/G-CSF-treated joint with moderate inflammatory features (200X). n ≥ 10 joints/group/experiment; representative experiment of three. * p < 0.05 compared to mBSA/saline controls.

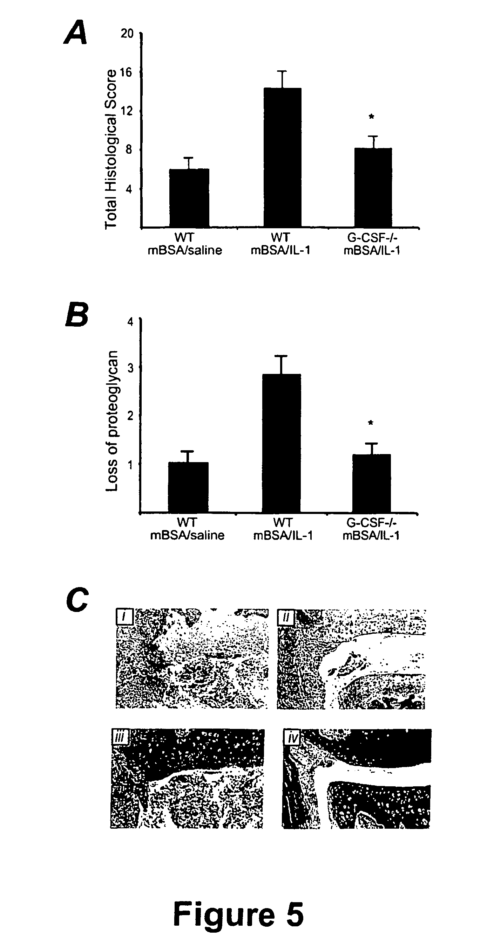

**Figure 5A-C** *G-CSF-*i/*-* mice are relatively resistant to mBSA/IL-1-induced arthritis. B6 and *G-CSF-*/*-* mice were treated i.a. with mBSA and s.c. with IL-1 or saline and histologically assessed at day 7. Histograms illustrate ***A*** Total histological severity scores and ***B*** cartilage proteoglycan loss (by safranin O staining). ***C*** Representative sections showing H&E (top panel) and safranin O (bottom panel) sections from mBSA/IL-1-treated (***i, iii***) B6 and (***ii, iv***) *G-CSF-*/*-* mice. n > 6 joints/group/experiment; representative of 3 experiments.

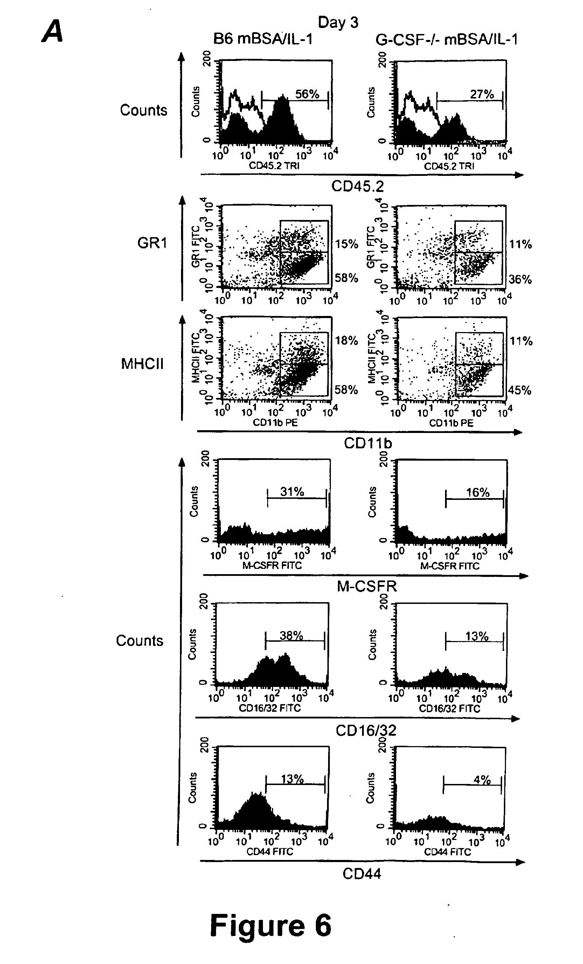

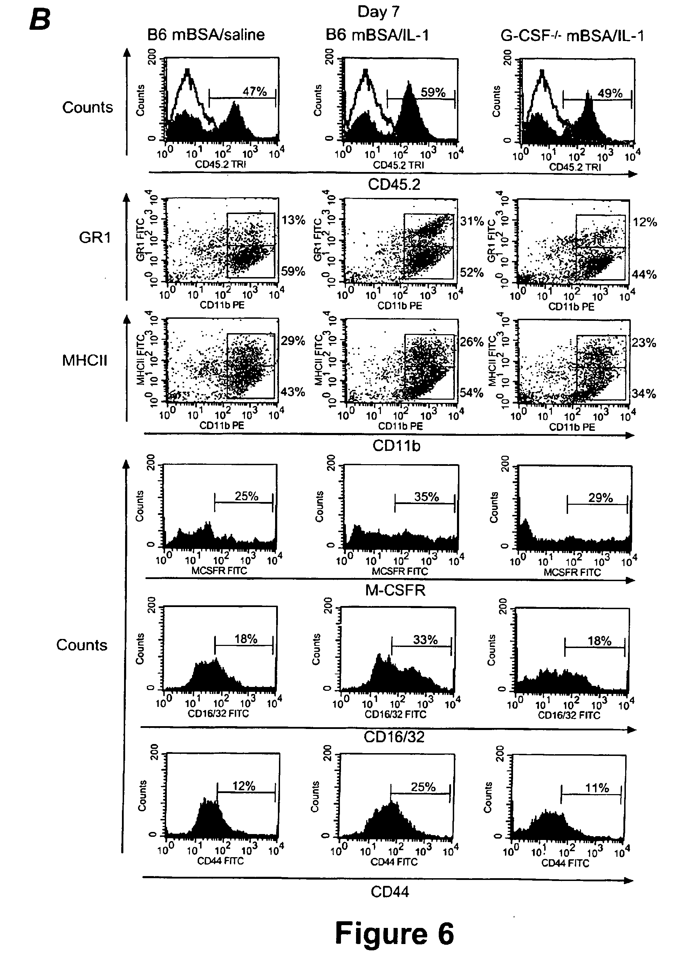

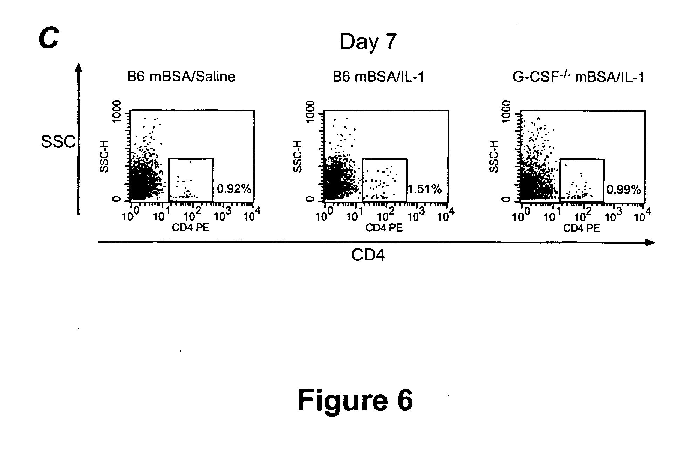

**Figure 6** shows representative FACS plots of leukocyte populations infiltrating the synovial tissue from B6 mBSA/saline, B6 mBSA/IL-1 and *G-CSF-*/*-* mBSA/IL-1 treated mice at days 3 and 7. Synovium was dissected at ***A*** day 3 and ***B*** day 7 from greater than 6 joints/group, dissociated in an enzyme cocktail and then stained for specified markers. In A & B, total infiltrating leukocytes were identified by staining with CD45.2 (***A,B;*** top panel). Only the CD45.2+ population was used for subsequent analyses. ***C*** Synovial digests from B6 mBSA/saline, B6 mBSA/IL-1 and *G-CSF-*/*-* mBSA/IL-1 treated mice were also adhered overnight and non-adherent cells stained for CD4 expression. Data are representative of 2 experiments.

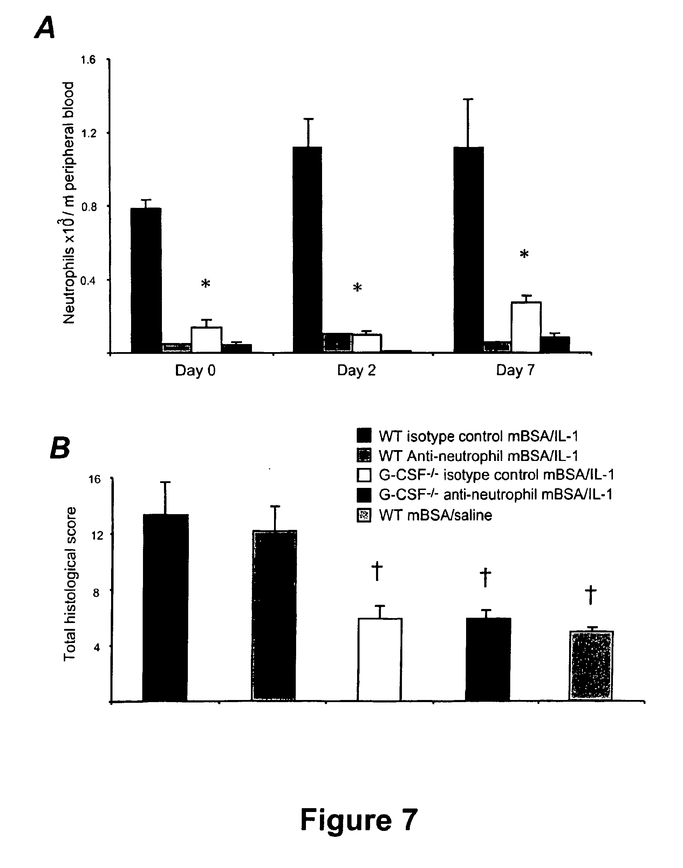

**Figure 7A and 7B** are graphical representations showing the effects of neutrophil depletion in acute arthritis in WT B6 and *G-CSF-*/*-* mice. Mice were treated prior to mBSA/IL-1-induced arthritis induction with a depleting mAb to neutrophils or isotype control. ***A*** Peripheral blood analysis on days 0, 2 and 7 of the acute arthritis model in WT and *G-CSF-*/*-* mice treated with neutrophil-depleting mAb or isotype control mAb. ***B*** Total histological scores of WT and *G-CSF-*/*-* mice treated with anti-neutrophil mAb or isotype control. n > 5 joints per group * P < 0.05 compared to WT isotype control mAb-treated mice neutrophil levels. † P < 0.05 compared to WT isotype control and anti-neutrophil mAb-treated control group total scores.

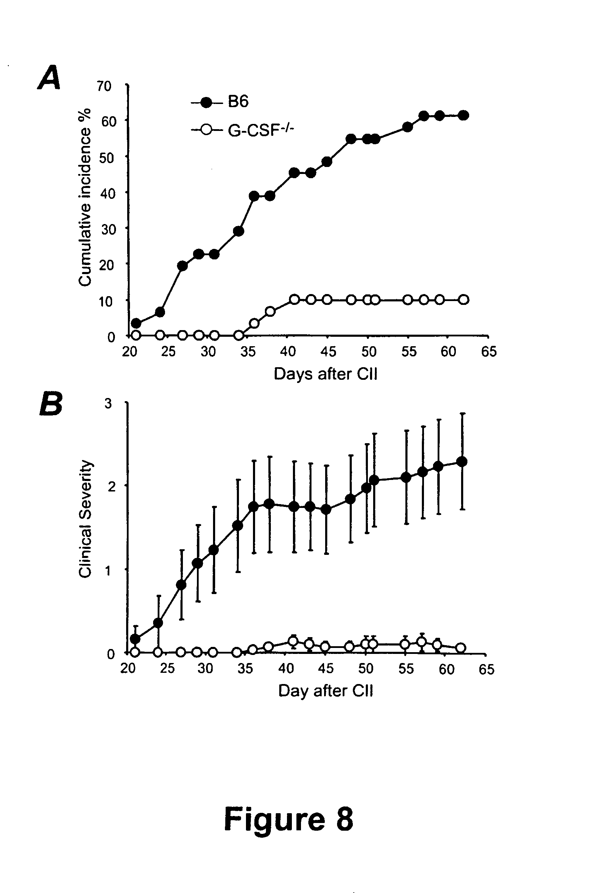

**Figure 8** graphical representations showing impaired CIA in *G-CSF-l-* mice compared to B6 mice. Mice were injected intra-dermally at the base of the tail with CII in CFA and boosted at day 21. Mice were clinically assessed for disease from day 21 with each paw being scored from 0 (normal) to three (severe); maximal score 12 (For details see EXAMPLE 8). ***A***. illustrates the cumulative incidence of disease. ***B***. The clinical severity of CIA in B6 and *G-CSF-*/*-* mice. Data are pooled from 3 experiments; n > 30 mice per group. * P < 0.001 B6 compared to *G-CSF-*/*-.*

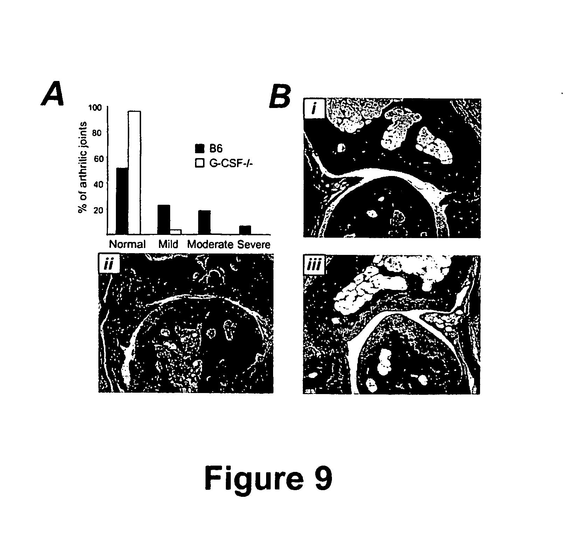

**Figure 9** shows results of histological assessment of CIA in B6 versus *G-CSF-*/*-* mice. Joints from four of the most severely clinically affected B6 and *G-CSF-*/*-* mice were scored from 0 to 3 for histopathology severity. ***A*** is a graphical representation of the percentage of normal, mild, moderate and severely affected joints. ***B*** shows representative H&E sections from *i* a non-arthritic B6 joint, *ii* a severely inflamed B6 CIA joint and *iii a* typical *G-CSF-*/*-* joint that exhibits no inflammation.

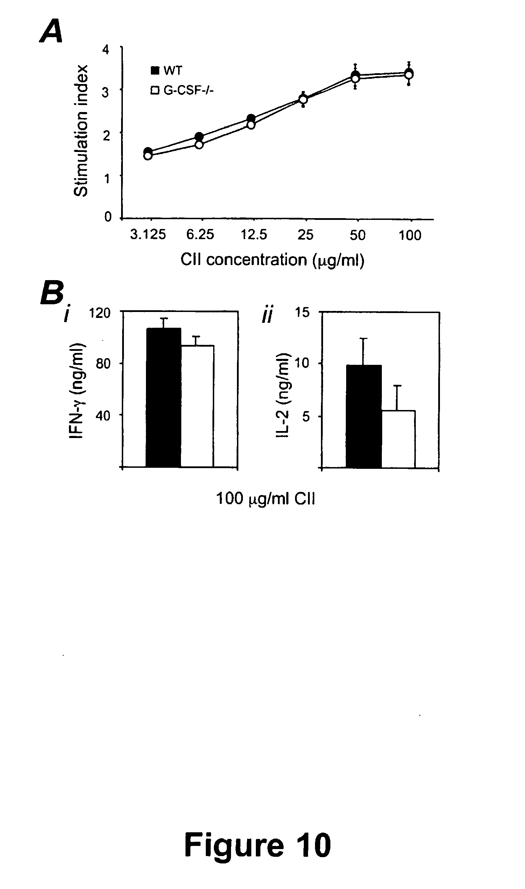

**Figure 10** are graphical representations showing T cell responses to CII *in vitro* in B6 and *G-CSF-*/*-* mice. Single inguinal LN suspensions were plated and stimulated with denatured CII. Cells were pulsed for the last 8 h with [³H] TdR and radioactive uptake measured to assess T cell proliferation. ***A***. Proliferative stimulation index in B6 and *G-CSF-*/*-* LN cells. ***B***. Supernatants taken from cultures at 64 h were assayed for levels of (i) IFN-γ and (ii) IL-2 by ELISA. n > 6 wells/sample.

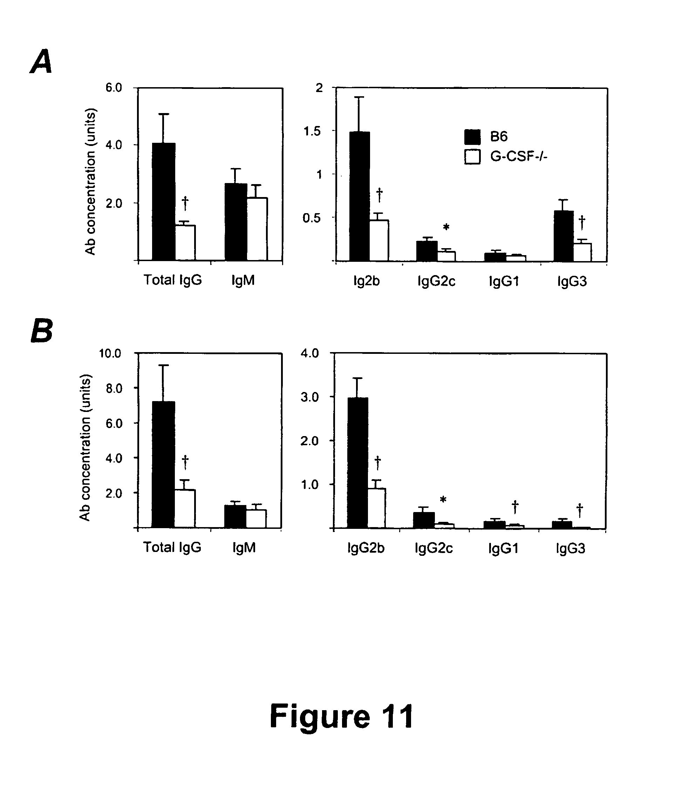

**Figure 11A and B** show anti-CII Abs in CIA-immunised B6 and *G-CSF-*/*-* mice. Serum was taken at ***A***. day 30 and ***B***. day 62 and analysed by ELISA for anti-CII Abs - total IgG, IgM and isotypes IgG2b, IgG2c, IgG1 and IgG3. Data are pooled from 3 experiments; n>30 samples per group. * P < 0.05, † P < 0.005.

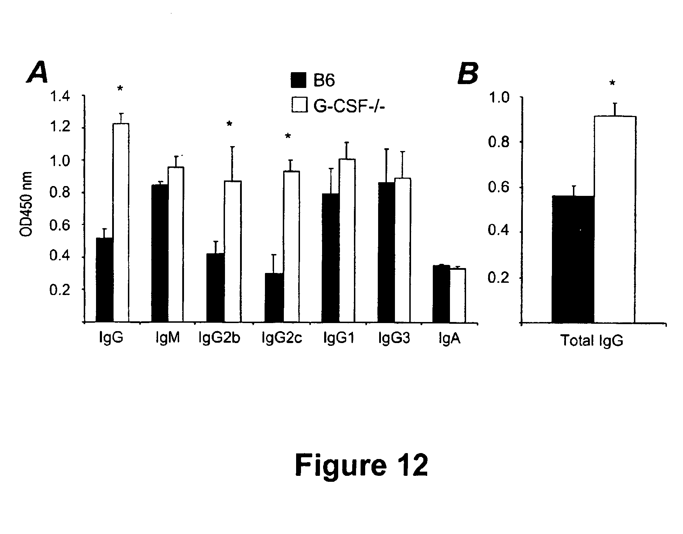

**Figure 12** shows graphical representation of basal Ig levels and levels of non-specific total IgG in naive and CII/CFA-immunised B6 and *G-CSF-*/*-* mice. ***A***. Naïve B6 and *G-CSF-*/*-* mice (n=6 mice/group) were bled and sera tested by ELISA for levels of circulating total IgG, IgM and isotypes (IgG2b, IgG2c, IgG1, IgG3 and IgA). ***B***. Day 62 sera from CIA-immunised B6 and *G-CSF-*/*-* mice was analysed for non-specific total IgG. Mice from 3 separate experiments are included; n ≥ 16 mice/group; * P < 0.05.

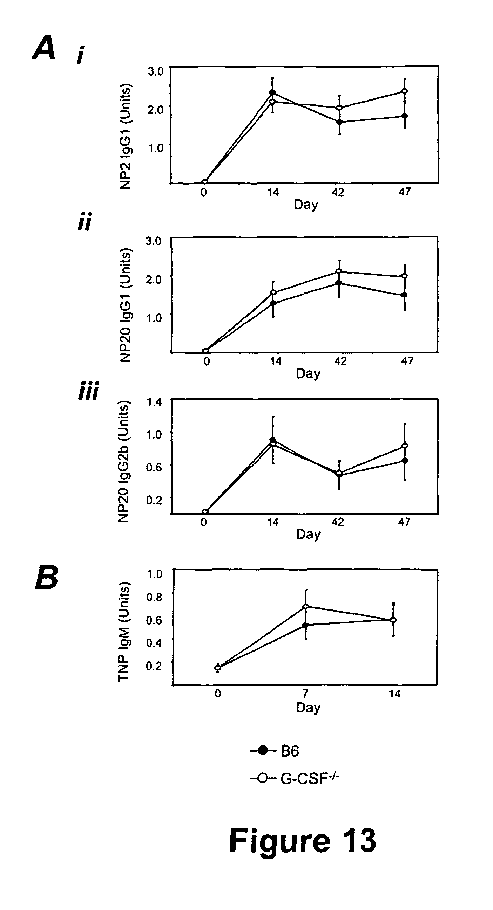

**Figure 13** shows Ab responses to T-dependent and T-independent antigens in *G-CSF-*/*-* mice compared to B6 mice. B6 and *G-CSF-*/*-* mice were challenged with the T-dependent Ag NP-KLH precipitated in alum or with the T-independent antigen DNP-Dextran in PBS and bled on specified days. The NP-KLH group were boosted at day 42. Sera were analysed by ELISA for assessment of ***A***. T-dependent NP-KLH response as assessed by the titres of *i* NP₂ (total) and *ii* NP₂₀ (high affinity)-specific IgGI, ***iii*** NP₂₀ Ig2b and ***B*** T-independent DNP-dextran anti-NP IgM. n > 4 mice per group.

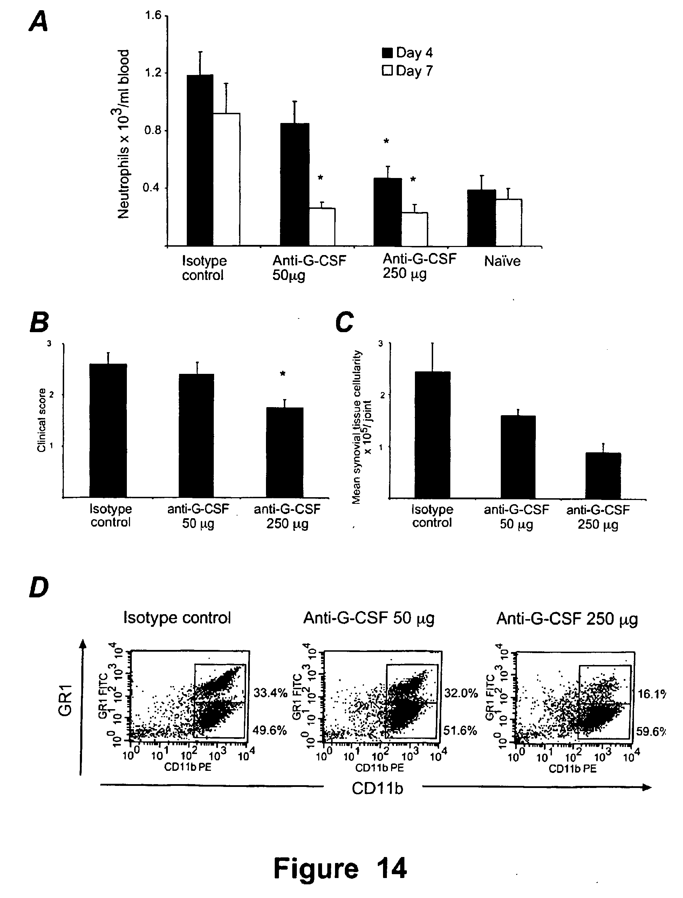

**Figure 14** shows effects of G-CSF neutralisation in the acute arthritis model. B6 mice were treated with neutralising anti-G-CSF (50 and 250 µg) or isotype control mAb on days 0, 1, 2, 3 and 5. Graphical representations of ***A***. peripheral blood neutrophil counts at days 4 and 7 (n=5) ***B**.* Mean clinical macroscopic score per joint (out of 4) (n=10) ***C***. mean synovial tissue cellularity per mouse joint (n=3). ***D*.** Dot plots showing infiltrating neutrophils (GR1*hi* CD11 b*hi*) and monocyte/macrophages (CD11b*hi* GR1*lo*). Data are representative of a single experiment; * P < 0.01.

### DETAILED DESCRIPTION OF THE PREFERRED EMBODIMENTS

The present invention is predicated in part on the further elucidation of the role of inflammatory cytokines in the inflammatory process. More particularly, the role of G-CSF is postulated to have an effect on inflammatory conditions such as chronic immune-mediated inflammation. In accordance with the present invention, therefore, inhibiting the activity of the inflammatory cytokine locally or systemically and/or down-regulating expression of a gene encoding an inflammatory cytokine is proposed to be useful in the treatment or prophylaxis of arthritis.

Accordingly, one aspect of the present invention contemplates a use of an agent which inhibits the activity of G-CSF or G-CSFR and/or which reduces the level of expression of a gene encoding said G-CSF or G-CSFR, in the manufacture of a medicament for the treatment or prophylaxis of arthritis in a subject.

The present invention is particularly directed to G-CSF and its homologs and derivatives. Reference to "G-CSF" or its full name "granulocyte-colony stimulating factor" includes it homologs and derivatives. A "homolog" or "derivative" includes polymorphic variants of G-CSF.

Accordingly, another aspect of the present invention provides an agent for the treatment and/or prophylaxis of arthritis in a subject, said agent being an agent which inhibits the activity of G-CSF or G-CSFR and/or which reduces the level of expression of the gene encoding G-CSF or G-CSFR.

As indicated above, reference to "G-CSF" includes its homologs and its derivatives.

Administration of the agent may be systemic or local. Local administration is particularly useful in the treatment of localized or inflammatory conditions such as arthritis.
However, as it is likely that G-CSF exerts effects on haemopoietic cells, systemic administration may be useful in modulating the immune system in general. Reference to "systemic" includes intra-articular, intravenous, intraperitoneal, subcutaneous and intrathecal administration as well as administration via oral, rectal and nasal routes.

Accordingly, in a preferred embodiment, the present invention contemplates a use of an agent which inhibits the activity of G-CSF or G-CSFR and/or which reduces the level of expression of the gene encoding G-CSF or G-CSFR in the manufacture of a medicament for the treatment and/or prophylaxis of arthritis in a subject.

In one embodiment, the arthritis is inflammatory arthritis, including rheumatoid arthritis.

The preferred animals are mammals such as humans and other primates, livestock animals (e. g. sheep, cows, horses, donkeys, pigs), laboratory test animals (e. g. rabbits, mice, hamsters, guinea pigs), companion animals (e. g. dogs, cats) and captive wild animals. Avian species include poultry birds (e. g. chickens, ducks, geese, turkeys, bantams), game birds (e. g. ducks, emus, pheasants) and caged avian birds. Humans are the most preferred animals of the primates. Horses are particularly preferred of the livestock animals.

In a preferred embodiment, therefore, the present invention provides a use of an agent which inhibits the activity of G-CSF or G-CSFR and/or which reduces the level of expression of the gene encoding G-CSF or G-CSFR in the manufacture of a medicament for the treatment and/or prophylaxis of arthritis in a human.

The agents may be proteinaceous, non-proteinaceous (e.g. chemical entities) or nucleic acid molecules.

Proteinaceous and non-proteinaceous molecules include peptides, polypeptides and proteins, small, intermediate or large chemical molecules as well as molecules identified from natural product screening or the screening of chemical libraries. Natural product screening includes the screening of extracts or samples from plants, microorganisms, soil river beds, coral, aquatic environments and extraterrestrial environments for molecules or groups of molecules which have an affect on G-CSF activity or the level of G-CSF gene expression. These molecules may also affect G-CSF/G-CSFR interaction.

One example of an agent is an antibody to G-CSF or G-CSFR or epitopes thereon. This could be used systemically or locally.

The use of monoclonal antibodies is particularly preferred because of the ability to produce them in large quantities and the homogeneity of the product. The preparation of hybridoma cell lines for monoclonal antibody production derived by fusing an immortal cell line and lymphocytes sensitized against the immunogenic preparation can be done by techniques which are well known to those who are skilled in the art. (See, for example, Douillard and Hoffman, Basic Facts about Hybridomas, in Compendium of Immunology Vol. II, ed. by Schwartz, 1981; Kohler and Milstein, Nature 256: 495-499, 1975; Kohler and Milstein, European Journal of Immunology 6: 511-519, 1976).

Another example of a useful agent is a soluble form of the G-CSFR which competes with G-CSF interaction with the membrane-associated G-CSFR.

Alternatively, agents can be screened for their ability to bind to G-CSF or G-CSFR-genetic materials. In one embodiment, G-CSF- or G-CSFR- encoding cDNA or genomic DNA or mRNA transcript or portion thereof such as an EST or SAGE tag is immobilized to a solid support such as a nanoparticle or microsphere. Potential agents are then brought into contact with the immobilized nucleic acid molecules and binding detected by change in radiation, emissions, atom excitation, mass and/or density.

Once identified, the agent is eluted off the nucleic acid molecule and characterized in more detail. For example, agents which bind to G-CSF/G-CSFR genetic material may inhibit expression (transcription and/or translation).

The present invention further contemplates using chemical analogs of G-CSF or G-CSFR as antagonists of G-CSF or its receptors. As indicated above, soluble G-CSF receptors may also be employed.

Chemical analogs contemplated herein include, but are not limited to, modifications of side chains, incorporation of unnatural amino acids and/or their derivatives during peptide, polypeptide or protein synthesis and the use of crosslinkers and other methods which impose conformational constraints on the proteinaceous molecule or their analogs.

Examples of side chain modifications contemplated by the present invention include modifications of amino groups such as by reductive alkylation by reaction with an aldehyde followed by reduction with NaBH₄; amidination with methylacetimidate; acylation with acetic anhydride; carbamoylation of amino groups with cyanate; trinitrobenzylation of amino groups with 2, 4, 6-trinitrobenzene sulphonic acid (TNBS); acylation of amino groups with succinic anhydride and tetrahydrophthalic anhydride; and pyridoxylation of lysine with pyridoxal-5-phosphate followed by reduction with NaBH₄.

The guanidine group of arginine residues may be modified by the formation of heterocyclic condensation products with reagents such as 2,3-butanedione, phenylglyoxal and glyoxal.

The carboxyl group may be modified by carbodiimide activation via O-acylisourea formation followed by subsequent derivitisation, for example, to a corresponding amide.

Sulphydryl groups may be modified by methods such as carboxymethylation with iodoacetic acid or iodoacetamide; performic acid oxidation to cysteic acid; formation of a mixed disulphides with other thiol compounds; reaction with maleimide, maleic anhydride or other substituted maleimide; formation of mercurial derivatives using 4-chloromercuribenzoate, 4-chloromercuriphenylsulphonic acid, phenylmercury chloride, 2-chloromercuri-4-nitrophenol and other mercurials; carbamoylation with cyanate at alkaline pH.

Tryptophan residues may be modified by, for example, oxidation with N-bromosuccinimide or alkylation of the indole ring with 2-hydroxy-5-nitrobenzyl bromide or sulphenyl halides. Tyrosine residues on the other hand, may be altered by nitration with tetranitromethane to form a 3-nitrotyrosine derivative.

Modification of the imidazole ring of a histidine residue may be accomplished by alkylation with iodoacetic acid derivatives or N-carbethoxylation with diethylpyrocarbonate.

Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids. A list of unnatural amino acid, contemplated herein is shown in Table 2.

**TABLE 2**

| Non-conventional amino acid | Code | Non-conventional amino acid | Code |
|---|---|---|---|
| α-aminobutyric acid | Abu | L-N-methylalanine | Nmala |
| α-amino-α-methylbutyrate | Mgabu | L-N-methylarginine | Nmarg |
| aminocyclopropane- | Cpro | L-N-methylasparagine | Nmasn |
| carboxylate | | L-N-methylaspartic acid | Nmasp |
| aminoisobutyric acid | Aib | L-N-methylcysteine | Nmcys |
| aminonorbornyl- | Norb | L-N-methylglutamine | Nmgln |
| carboxylate | | L-N-methylglutamic acid | Nmglu |
| cyclohexylalanine | Chexa | L-Nmethylhistidine | Nmhis |
| cyclopentylalanine | Cpen | L-N-methylisolleucine | Nmile |
| D-alanine | Dal | L-N-methylleucine | Nmleu |
| D-arginine | Darg | L-N-methyllysine | Nmlys |
| D-aspartic acid | Dasp | L-N-methylmethionine | Nmmet |
| D-cysteine | Dcys | L-N-methylnorleucine | Nmnle |
| D-glutamine | Dgln | L-N-methylnorvaline | Nmnva |
| D-glutamic acid | Dglu | L-N-methylornithine | Nmorn |
| D-histidine | Dhis | L-N-methylphenylalanine | Nmphe |
| D-isoleucine | Dile | L-N-methylproline | Nmpro |
| D-leucine | Dleu | L-N-methylserine | Nmser |
| D-lysine | Dlys | L-N-methylthreonine | Nmthr |
| D-methionine | Dmet | L-N-methyltryptophan | Nmtrp |
| D-omithine | Dom | L-N-methyltyrosine | Nmtyr |
| D-phenylalanine | Dphe | L-N-methylvaline | Nmval |
| D-proline | Dpro | L-N-methylethylglycine | Nmetg |
| D-serine | Dser | L-N-methyl-t-butylglycine | Nmtbug |
| D-threonine | Dthr | L-norleucine | Nle |
| D-tryptophan | Dtrp | L-norvaline | Nva |
| D-tyrosine | Dtyr | α-methyl-aminoisobutyrate | Maib |
| D-valine | Dval | α-methyl-γ-aminobutyrate | Mgabu |
| D-α-methylalanine | Dmala | α-methylcyclohexylalanine | Mchexa |
| D-α-methylarginine | Dmarg | α-methylcylcopentylalanine | Mcpen |
| D-α-methylasparagine | Dmasn | α-methyl-α-napthylalanine | Manap |
| D-α-methylaspartate | Dmasp | α-methylpenicillamine | Mpen |
| D-α-methylcysteine | Dmcys | N-(4-aminobutyl)glycine | Nglu |
| D-α-methylglutamine | Dmgln | N-(2-aminoethyl)glycine | Naeg |
| D-α-methylhistidine | Dmhis | N-(3-aminopropyl)glycine | Norn |
| D-α-methylisoleucine | Dmile | N-amino-α-methylbutyrate | Nmaabu |
| D-α-methylleucine | Dmleu | α-napthylalanine | Anap |
| D-α-methyllysine | Dmlys | N-benzylglycine | Nphe |
| D-α-methylmethionine | Dmmet | N-(2-carbaMylethyl)glycine | Ngln |
| D-α-methylomithine | Dmom | N-(carbamylmethyl)glycine | Nasn |
| D-α-methylphenylalanine | Dmphe | N-(2-carboxyethyl)glycine | Nglu |
| D-α-methylproline | Dmpro | N-(carboxymethyl)glycine | Nasp |
| D-α-methylserine | Dmser | N-cyclobutylglycine | Ncbut |
| D-α-methylthreonine | Dmthr | N-cycloheptylglycine | Nchep |
| D-α-methyltryptophan | Dmtrp | N-cyclohexylglycine | Nchex |
| D-α-methyltyrosine | Dmty | N-cyclodecylglycine | Ncdec |
| D-α-methylvaline | Dmval | N-cylcododecylglycine | Ncdod |
| D-N-methylalanine | Dnmala | N-cyclooctylglycine | Ncoct |
| D-N-methylarginine | Dnmarg | N-cyclopropylglycine | Ncpro |
| D-N-methylasparagine | Dnmasn | N-cycloundecylglycine | Ncund |
| D-N-methylaspartate | Dnmasp | N-(2,2-diphenylethyl)glycine | Nbhm |
| D-N-methylcysteine | Dnmcys | N-(3,3-diphenylpropyl)glycine | Nbhe |
| D-N-methylglutamine | Dnmgln | N-(3-guanidinopropyl)glycine | Narg |
| D-N-methylglutamate | Dnmglu | N-(1-hydroxyethyl)glycine | Nthr |
| D-N-methylhistidine | Dnmhis | N-(hydroxyethyl))glycine | Nser |
| D-N-methylisoleucine | Dnmile | N-(imidazolylethyl))glycine | Nhis |
| D-N-methylleucine | Dnmleu | N-(3-indolylyethyl)glycine | Nhtrp |
| D-N-methyllysine | Dnmlys | N-methyl-γ-aminobutyrate | Nmgabu |
| N-methylcyclohexylalanine | Nmchexa | D-N-methylmethionine | Dnmmet |
| D-N-methylornithine | Dnmom | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nval |
| D-N-methyltyrosine | Dnmtyr | N-methyla-napthylalanine | Nmanap |
| D-N-methylvaline | Dnmval | N-methylpenicillamine | Nmpen |
| γ-aminobutyric acid | Gabu | N-(*p*-hydroxyphenyl)glycine | Nhtyr |
| L-t-butylglycine | Thug | N-(thiomethyl)glycine | Ncys |
| L-ethylglycine | Etg | penicillamine | Pen |
| L-homophenylalanine | Hphe | L-α-methylalanine | Mala |
| L-α-methylarginine | Marg | L-α-methylasparagine | Masn |
| L-α-methylaspartate | Masp | L-α-methyl-t-butylglycine | Mtbug |
| L-α-methylcysteine | Mcys | L-methylethylglycine | Metg |
| L-α-methylglutamine | Mgln | L-α-methylglutamate | Mglu |
| L-α-methylhistidine | Mhis | L-α-methylhomophenylalanine | Mhphe |
| L-α-methylisoleucine | Mile | N-(2-methylthioethyl)glycine | Nmet |
| L-α-methylleucine | Mleu | L-α-methyllysine | Mlys |
| L-α-methylmethionine | Mmet | L-α-methylnorleucine | Mnle |
| L-α-methylnorvaline | Mnva | L-α-methylomithine | Mom |
| L-α-methylphenylalanine | Mphe | L-α-methylproline | Mpro |
| L-α-methylserine | Mser | L-α-methylthreonine | Mthr |
| L-α-methyltryptophan | Mtrp | L-α-methyltyrosine | Mtyr |
| L-α-methylvaline | Mval | L-N-methylhomophenylalanine | Nmhphe |
| N-(N-(2,2-diphenylethyl) | Nnbhm | N-(N-(3,3-diphenylpropyl) | Nnbhe |
| carbamylmethyl)glycine | | carbamylmethyl)glycine | |
| 1-carboxy-1-(2,2-diphenyl-ethylamino)cyclopropane | Nmbc | | |

Crosslinkers can be used, for example, to stabilize 3D conformations, using homo-bifunctional crosslinkers such as the bifunctional imido esters having (CH₂)ₙ spacer groups with n=1 to n=6, glutaraldehyde, N-hydroxysuccinimide esters and hetero-bifunctional reagents which usually contain an amino-reactive moiety such as N-hydroxysuccinimide and another group specific-reactive moiety such as maleimido or dithio moiety (SH) or carbodiimide (COOH). In addition, peptides can be conformationally constrained by, for example, incorporation of C_{α} and N_{α}-methylamino acids, introduction of double bonds between C_{α} and C_{β} atoms of amino acids and the formation of cyclic peptides or analogs by introducing covalent bonds such as forming an amide bond between the N and C termini, between two side chains or between a side chain and the N or C terminus.

Nucleic acid molecules such as RNA or DNA are particularly useful for inducing gene silencing by antisense- or sense-mediated mechanisms. Sense-mediated gene silencing is also referred to as co-suppression and involves a range of mechanisms including the induction of RNAi.

The terms "nucleic acids", "nucleotide" and "polynucleotide" include RNA, cDNA, genomic DNA, synthetic forms and mixed polymers, both sense and antisense strands, and may be chemically or biochemically modified or may contain non-natural or derivatized nucleotide bases, as will be readily appreciated by those skilled in the art. Such modifications include, for example, labels, methylation, substitution of one or more of the naturally occurring nucleotides with an analog (such as the morpholine ring), internucleotide modifications such as uncharged linkages (e.g. methyl phosphonates, phosphotriesters, phosphoamidates, carbamates, etc.), charged linkages (e.g. phosphorothioates, phosphorodithioates, etc.), pendent moieties (e.g. polypeptides), intercalators (e.g. acridine, psoralen, etc.), chelators, alkylators and modified linkages (e.g. α-anomeric nucleic acids, etc.). Also included are synthetic molecules that mimic polynucleotides in their ability to bind to a designated sequence *via* hydrogen binding and other chemical interactions. Such molecules are known in the art and include, for example, those in which peptide linkages substitute for phosphate linkages in the backbone of the molecule.

Antisense polynucleotide sequences, for example, are useful in silencing transcripts of the G-CSF genetic sequence or the G-CSFR genetic sequence. Furthermore, polynucleotide vectors containing all or a portion of the G-CSF gene locus may be placed under the control of a promoter in either the sense or antisense orientation and introduced into a cell. Expression of such a sense or antisense construct within a cell interferes with target transcription and/or translation. Furthermore, co-suppression (i.e. using sense-suppresion) and mechanisms to induce RNAi or siRNA may also be employed. Alternatively, antisense or sense molecules may be directly administered. In this latter embodiment, the antisense or sense molecules may be formulated in a composition and then administered by any number of means to target cells.

A variation on antisense and sense molecules involves the use of morpholinos, which are oligonucleotides composed of morpholine nucleotide derivatives and phosphorodiamidate linkages (for example, Summerton and Weller, Antisense and Nucleic Acid Drug Development 7: 187-195, 1997). Such compounds are injected into non-human embryos and the effect of interference with mRNA is observed.

In one embodiment, the present invention employs compounds such as oligonucleotides and similar species for use in modulating the function or effect of nucleic acid molecules encoding G-CSF or G-CSFR, i.e. the oligonucleotides induce transcriptional or post-transcriptional gene silencing. This is accomplished by providing oligonucleotides which specifically hybridize with one or more nucleic acid molecules encoding the G-CSF or G-CSFR. The oligonucleotides may be provided directly to a cell or generated within the cell. As used herein, the terms "target nucleic acid" and "nucleic acid molecule encoding G-CSF or G-CSFR" have been used for convenience to encompass the encoding DNA, RNA (including pre-mRNA and mRNA or portions thereof) transcribed from such DNA, and also cDNA derived from such RNA. The hybridization of a compound of the subject invention with its target nucleic acid is generally referred to as "antisense". Consequently, the preferred mechanism believed to be included in the practice of some preferred embodiments of the invention is referred to herein as "antisense inhibition." Such antisense inhibition is typically based upon hydrogen bonding-based hybridization of oligonucleotide strands or segments such that at least one strand or segment is cleaved, degraded, or otherwise rendered inoperable. In this regard, it is presently preferred to target specific nucleic acid molecules and their functions for such antisense inhibition.

The functions of DNA to be interfered with can include replication and transcription. Replication and transcription, for example, can be from an endogenous cellular template, a vector, a plasmid construct or otherwise. The functions of RNA to be interfered with can include functions such as translocation of the RNA to a site of protein translation, translocation of the RNA to sites within the cell which are distant from the site of RNA synthesis, translation of protein from the RNA, splicing of the RNA to yield one or more RNA species, and catalytic activity or complex formation involving the RNA which may be engaged in or facilitated by the RNA.

In the context of this invention, "hybridization" means the pairing of complementary strands of oligomeric compounds. In the present invention, the preferred mechanism of pairing involves hydrogen bonding, which may be Watson-Crick, Hoogsteen or reversed Hoogsteen hydrogen bonding, between complementary nucleoside or nucleotide bases (nucleobases) of the strands of oligomeric compounds. For example, adenine and thymine are complementary nucleobases which pair through the formation of hydrogen bonds. Hybridization can occur under varying circumstances.

An antisense compound is specifically hybridizable when binding of the compound to the target nucleic acid interferes with the normal function of the target nucleic acid to cause a loss of activity, and there is a sufficient degree of complementarity to avoid non-specific binding of the antisense compound to non-target nucleic acid sequences under conditions in which specific binding is desired.

"Complementary" as used herein, refers to the capacity for precise pairing between two nucleobases of an oligomeric compound. For example, if a nucleobase at a certain position of an oligonucleotide (an oligomeric compound), is capable of hydrogen bonding with a nucleobase at a certain position of a target nucleic acid, said target nucleic acid being a DNA, RNA, or oligonucleotide molecule, then the position of hydrogen bonding between the oligonucleotide and the target nucleic acid is considered to be a complementary position. The oligonucleotide and the further DNA, RNA, or oligonucleotide molecule are complementary to each other when a sufficient number of complementary positions in each molecule are occupied by nucleobases which can hydrogen bond with each other. Thus, "specifically hybridizable" and "complementary" are terms which are used to indicate a sufficient degree of precise pairing or complementarity over a sufficient number of nucleobases such that stable and specific binding occurs between the oligonucleotide and a target nucleic acid.

According to the present invention, compounds include antisense oligomeric compounds, antisense oligonucleotides, ribozymes, external guide sequence (EGS) oligonucleotides, alternate splicers, primers, probes, and other oligomeric compounds which hybridize to at least a portion of the target nucleic acid. As such, these compounds may be introduced in the form of single-stranded, double-stranded, circular or hairpin oligomeric compounds and may contain structural elements such as internal or terminal bulges or loops. Once introduced to a system, the compounds of the invention may elicit the action of one or more enzymes or structural proteins to effect modification of the target nucleic acid. One non-limiting example of such an enzyme is RNAse H, a cellular endonuclease which cleaves the RNA strand of an RNA:DNA duplex. It is known in the art that single-stranded antisense compounds which are "DNA-like" elicit RNAse H. Activation of RNase H, therefore, results in cleavage of the RNA target, thereby greatly enhancing the efficiency of oligonucleotide-mediated inhibition of gene expression. Similar roles have been postulated for other ribonucleases such as those in the RNase III and ribonuclease L family of enzymes.

While the preferred form of antisense compound is a single-stranded antisense oligonucleotide, in many species the introduction of double-stranded structures, such as double-stranded RNA (dsRNA) molecules, has been shown to induce potent and specific antisense-mediated reduction of the function of a gene or its associated gene products.

In the context of the subject invention, the term "oligomeric compound" refers to a polymer or oligomer comprising a plurality of monomeric units. In the context of this invention, the term "oligonucleotide" refers to an oligomer or polymer of ribonucleic acid (RNA) or deoxyribonucleic acid (DNA) or mimetics, chimeras, analogs and homologs thereof. This term includes oligonucleotides composed of naturally occurring nucleobases, sugars and covalent internucleoside (backbone) linkages as well as oligonucleotides having non-naturally occurring portions which function similarly. Such modified or substituted oligonucleotides are often preferred over native forms because of desirable properties such as, for example, enhanced cellular uptake, enhanced affinity for a target nucleic acid and increased stability in the presence of nucleases.

While oligonucleotides are a preferred form of the compounds of this invention, the present invention comprehends other families of compounds as well, including but not limited to oligonucleotide analogs and mimetics such as those herein described.

The open reading frame (ORF) or "coding region" which is known in the art to refer to the region between the translation initiation codon and the translation termination codon, is a region which may be effectively targeted. Within the context of the present invention, one region is the intragenic region encompassing the translation initiation or termination codon of the open reading frame (ORF) of a gene.

Other target regions include the 5' untranslated region (5'UTR), known in the art to refer to the portion of an mRNA in the 5' direction from the translation initiation codon, and thus including nucleotides between the 5' cap site and the translation initiation codon of an mRNA (or corresponding nucleotides on the gene), and the 3' untranslated region (3'UTR), known in the art to refer to the portion of an mRNA in the 3' direction from the translation termination codon, and thus including nucleotides between the translation termination codon and 3' end of an mRNA (or corresponding nucleotides on the gene). The 5' cap site of an mRNA comprises an N7-methylated guanosine residue joined to the 5'-most residue of the mRNA *via* a 5'-5' triphosphate linkage. The 5' cap region of an mRNA is considered to include the 5' cap structure itself as well as the first 50 nucleotides adjacent to the cap site. It is also preferred to target the 5' cap region.

Although some eukaryotic mRNA transcripts are directly translated, many contain one or more regions, known as "introns", which are excised from a transcript before it is translated. The remaining (and, therefore, translated) regions are known as "exons" and are spliced together to form a continuous mRNA sequence. Targeting splice sites, i.e. intron-exon junctions or exon-intron junctions, may also be particularly useful in situations where aberrant splicing is implicated in disease, or where an overproduction of a particular splice product is implicated in disease. Aberrant fusion junctions due to rearrangements or deletions are also preferred target sites. mRNA transcripts produced via the process of splicing of two (or more) mRNAs from different gene sources are known as "fusion transcripts". It is also known that introns can be effectively targeted using antisense compounds targeted to, for example, DNA or pre-mRNA.

As is known in the art, a nucleoside is a base-sugar combination. The base portion of the nucleoside is normally a heterocyclic base. The two most common classes of such heterocyclic bases are the purines and the pyrimidines. Nucleotides are nucleosides that further include a phosphate group covalently linked to the sugar portion of the nucleoside. For those nucleosides that include a pentofuranosyl sugar, the phosphate group can be linked to either the 2', 3' or 5' hydroxyl moiety of the sugar. In forming oligonucleotides, the phosphate groups covalently link adjacent nucleosides to one another to form a linear polymeric compound. In turn, the respective ends of this linear polymeric compound can be further joined to form a circular compound, however, linear compounds are generally preferred. In addition, linear compounds may have internal nucleobase complementarity and may, therefore, fold in a manner as to produce a fully or partially double-stranded compound. Within oligonucleotides, the phosphate groups are commonly referred to as forming the internucleoside backbone of the oligonucleotide. The normal linkage or backbone of RNA and DNA is a 3' to 5' phosphodiester linkage.

For topical delivery of antisense compounds, these oligonucleotides may contain modified backbones or non-natural internucleoside linkages. As defined in this specification, oligonucleotides having modified backbones include those that retain a phosphorus atom in the backbone and those that do not have a phosphorus atom in the backbone. For the purposes of this specification, and as sometimes referenced in the art, modified oligonucleotides that do not have a phosphorus atom in their internucleoside backbone can also be considered to be oligonucleosides.

Preferred modified oligonucleotide backbones containing a phosphorus atom therein include, for example, phosphorothioates, chiral phosphorothioates, phosphorodithioates, phosphotriesters, aminoalkylphosphotriesters, methyl and other alkyl phosphonates including 3'-alkylene phosphonates, 5'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, selenophosphates and boranophosphates having normal 3'-5' linkages, 2'-5' linked analogs of these, and those having inverted polarity wherein one or more internucleotide linkages is a 3' to 3', 5' to 5' or 2' to 2' linkage. Preferred oligonucleotides having inverted polarity comprise a single 3' to 3' linkage at the 3'-most internucleotide linkage i.e. a single inverted nucleoside residue which may be abasic (the nucleobase is missing or has a hydroxyl group in place thereof). Various salts, mixed salts and free acid forms are also included.

In an alternative embodiment, genetic constructs including DNA "vaccines" are used to generate antisense or sense molecules mammalian cells. Furthermore, many of the preferred features described above are appropriate for sense nucleic acid molecules.

Agents identified in accordance with the present invention are conveniently supplied in pharmaceutical compositions.

Readily conceivable by one of skill in the art in light of the foregoing disclosure will be a composition comprising a modulator of G-CSF activity or the interaction between G-CSF and G-CSFR or a modulator of expression of G-CSF/G-CSFR, said composition further comprising one or more pharmaceutically acceptable carriers and/or diluents.

Composition forms suitable for injectable use include sterile aqueous solutions (where water soluble) and sterile powders for the extemporaneous preparation of sterile injectable solutions. It must be stable under the conditions of manufacture and storage and must be preserved against the contaminating action of microorganism such as bacteria and fungi. The carrier can be a solvent or dilution medium comprising, for example, water, ethanol, polyol (for example, glycerol, propylene glycol and liquid polyethylene glycol, and the like), suitable mixtures thereof and vegetable oils. The proper fluidity can be maintained, for example, by the use of superfactants. The preventions of the action of microorganism can be brought about by various anti-bacterial and anti-fungal agents, for example, parabens, chlorobutanol, phenol, sorbic acid, thirmerosal and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compounds in the required amount in the appropriate solvent with the active ingredient and optionally other active ingredient as required, followed by filtered sterilization or other appropriate means of sterilization. In the case of sterile powders for the preparation of sterile injectable solutions, suitable methods of preparation include vacuum drying and the freeze-drying technique which yield a powder of active ingredient plus any additionally desired ingredient.

When the modulator is suitably protected, it may be orally administered, for example, with an inert diluent or with an assimilable edible carrier, or it may be enclosed in hard or soft shell gelatin capsule, or it may be compressed into tablets, or it may be incorporated directly with the food of the diet or administered via breast milk. For oral therapeutic administration, the active ingredient may be incorporated with excipients and used in the form of ingestible tablets, buccal tablets, troches, capsules, elixirs, suspensions, syrups, wafers and the like. Such compositions and preparations should contain at least 1% by weight of modulator. The percentage of the compositions and preparations may, of course, be varied and may conveniently be between about 5 to about 80% of the weight of the unit. The amount of modulator in such therapeutically useful compositions is such that a suitable dosage will be obtained. Preferred compositions or preparations according to the present invention are prepared so that an oral dosgae unit form contains between about 0.1 µg and 200 mg of modulator. Alternative dosage amounts include from about 1 µg to about 1000 mg and from about 10 µg to about 500 mg. These dosages may be per individual or per kg body weight. Administration may be per hour, day, week, month or year.

The tablets, troches, pills, capsules, creams and the like may also contain the components as listed hereafter. A binder such as gum, acacia, corn starch or gelatin; excipients such as dicalcium phosphate; a disintegrating agent such as com starch, potato starch, alginic acid and the like; a lubricant such as magnesium stearate; and a sweetening agent such as sucrose, lactose or saccharin may be added or a flavouring agent such as peppermint, oil of wintergreen or cherry flavouring. When the dosage unit form is a capsule, it may contain, in addition to materials of the above type, a liquid carrier. Various other materials may be present as coatings or to otherwise modify the physical form of the dosage unit. For instance, tablets, pills or capsules may be coated with shellac, sugar or both. A syrup or elixir may contain the active compound, sucrose as a sweetening agent, methyl and propylparabens as preservatives, a dye and flavouring such as cherry or orange flavour. Of course, any material used in preparing any dosage unit form should be pharmaceutically pure and substantially non-toxic in the amounts employed. In addition, the active compound(s) may be incorporated into sustained-release preparations and formulations.

Pharmaceutically acceptable carriers and/or diluents include any and all solvents, dispersion media, coatings, anti-bacterial and anti-fungal agents, isotonic and absorption delaying agents and the like. The use of such media and agents for pharmaceutical active substances is well known in the art and except insofar as any conventional media or agent is incompatible with the modulator, their use in the therapeutic compositions is contemplated. Supplementary active compounds can also be incorporated into the compositions.

Whilst administration may be by any means, intra-articular or subcutaneous administration is particularly preferred for the treatment of arthritis.

One skilled in the art can in light of the foregoing envisage that such a composition could also comprise genetic molecules such as a vector capable of transfecting target cells where the vector carries a nucleic acid molecule capable of encoding a modulator, when the modulator is a proteinaceous molecule. The vector could, for example, be a viral vector. In this regard, one can conceive a range of gene therapies including isolating certain cells, genetically manipulating and returning the cell to the same subject or to a genetically related or similar subject.

The present invention further provides an *in vivo* non-therapeutic method which uses a non-human animal model for screening for agents capable of inhibiting the activity of G-CSF and/or inhibiting the interaction of G-CSF with G-CSFR, said method comprising administering a putative inhibitory agent to a genetically modified non-human animal which has been modified to produce low levels of G-CSF or G-CSFR relative to a non-genetically modified animal of the same species, wherein said agent is identified as having interactivity with G-CSF or G-CSFR by the agent having a detectable physiological effect in a wild-type animal of the same species, but a reduced effect in an animal which exhibits reduced expression of G-CSF and/or G-CSFR. Furthermore, in animals with reduced levels of G-CSF, other cytokines or endogenous molecules may emerge to compensate G-CSF's absence. These then become targets for further therapeutic molecules.

For use in this aspect, a genetically modified animal may be constructed wherein said animal produces low amounts of G-CSF or G-CSFR. The genetically modified non-human animal may be a mouse, rat, guinea pig, rabbit, pig, sheep or goat.

In connection with such a genetically modified non-human animal, one skilled in the art could in light of the foregoing disclosure envisage a targeting vector useful for inactivating a gene encoding G-CSF or G-CSFR, said targeting vector comprising two segments of genetic material encoding said G-CSF or G-CSFR flanking a positive selectable marker wherein said targeting vector could be transfected into non-human embryonic stem (ES) cells and the marker selected to generate a non-human ES cell in which the gene encoding said G-CSF or G-CSFR is inactivated by homologous recombination.

Such non-human ES cells could be from mice, rats, guinea pigs, pigs, sheep or goats.

Such a targeting vector could be used in the manufacture of a genetically modified non-human animal substantially incapable of producing G-CSF or G-CSFR.

The vector could be DNA. A selectable marker in the targeting vector would allow for selection of targeted cells that have stably incorporated the targeting DNA. This is especially useful when employing relatively low efficiency transformation techniques such as electroporation, calcium phosphate precipitation and liposome fusion where typically fewer than 1 in 1000 cells will have stably incorporated the exogenous DNA. Using high efficiency methods, such as microinjection into nuclei, typically from 5-25% of the cells will have incorporated the targeting DNA; and it is, therefore, feasible to screen the targeted cells directly without the necessity of first selecting for stable integration of a selectable marker. Either isogenic or non-isogenic DNA could be employed.

Examples of selectable markers include genes conferring resistance to compounds such as antibiotics, genes conferring the ability to grow on selected substrates, genes encoding proteins that produce detectable signals such as luminescence. A wide variety of such markers are known and available, including, for example, antibiotic resistance genes such as the neomycin resistance gene *(neo)* and the hygromycin resistance gene (*hyg*). Selectable markers also include genes conferring the ability to grow on certain media substrates such as the *tk* gene (thymidine kinase) or the *hprt* gene (hypoxanthine phosphoribosyltransferase) which confer the ability to grow on HAT medium (hypoxanthine, aminopterin and thymidine); and the bacterial *gpt* gene (guanine/xanthine phosphoribosyltransferase) which allows growth on MAX medium (mycophenolic acid, adenine and xanthine). Other selectable markers for use in mammalian cells and plasmids carrying a variety of selectable markers are described in Sambrook et al., Molecular Cloning - A Laboratory Manual, Cold Spring Harbour, New York, USA, 1990.

The preferred location of the marker gene in the targeting construct would depend on the aim of the gene targeting. For example, if the aim is to disrupt target gene expression, then the selectable marker can be cloned into targeting DNA corresponding to coding sequence in the target DNA. Alternatively, if the aim was to express an altered product from the target gene, such as a protein with an amino acid substitution, then the coding sequence could be modified to code for the substitution, and the selectable marker could be placed outside of the coding region, for example, in a nearby intron.

The selectable marker could depend on its own promoter for expression and the marker gene could be derived from a very different organism than the organism being targeted (e.g. prokaryotic marker genes used in targeting mammalian cells).
However, it would be preferable to replace the original promoter with transcriptional machinery known to function in the recipient cells. A large number of transcriptional initiation regions are available for such purposes including, for example, metallothionein promoters, thymidine kinase promoters, β-actin promoters, immunoglobulin promoters, SV40 promoters and human cytomegalovirus promoters. A widely used example is the pSV2-neo plasmid which has the bacterial neomycin phosphotransferase gene under control of the SV40 early promoter and confers in mammalian cells resistance to G418 (an antibiotic related to neomycin). A number of other variations could be employed to enhance expression of the selectable markers in animal cells, such as the addition of a poly (A) sequence and the addition of synthetic translation initiation sequences. Both constitutive and inducible promoters could be used.

The DNA could be modified by homologous recombination. The target DNA could be in any organelle of the animal cell including the nucleus and mitochondria and could be an intact gene, an exon or intron, a regulatory sequence or any region between genes.

Homologous DNA is a DNA sequence that is at least 70% identical with a reference DNA sequence. An indication that two sequences are homologous is that they will hybridize with each other under stringent conditions (Sambrook *et al.,* 1990, *supra*).

The present invention further contemplates co-suppression (i.e, sense suppression) and antisense suppression to down-regulate expression of G-CSF or G-CSFR in the context of the aforementioned use of an agent in the manufacture of a medicament for the treatment or prophylaxis of arthritis, or for treatment or prophylaxis of arthritis. This could also occur in a target test non-human animal such as to generate a disease model, in which case the genetically modified non-human, animals may also produce larger amounts of G-CSF or G-CSFR. For example, over expression of normal G-CSF or G-CSFR may produce dominant negative effects and may become useful disease models.

Accordingly, one skilled in the art may contemplate a genetically modified non-human animal over-expressing a genetic sequence encoding G-CSF or G-CSFR.

A genetically modified non-human animal includes a transgenic animal, or a "knock-out" or "knock-in" animal.

The present invention is further described by the following non-limiting Examples. C57BL/6 (B6; wild type, [WT]) mice were obtained from the Walter and Eliza Hall Institute (WEHI) Animal Supplies (Victoria, Australia). G-CSF-deficient (G-CSF^{-/-}) mice were obtained from the Ludwig Institute for Cancer Research, Victoria, Australia and were produced by targeted disruption of the *Cysf3* gene in 129/OLA embryonic stem (ES) cells, which were injected into B6 blastocysts (Lieschke et al., Blood 84: 1737-1746, 1994). Mice were backcrossed greater than twenty generations onto the B6 background. All mice were ≥ 8 weeks of age at the time of experimentation, were fed standard rodent chow and water *ad libitum* and were housed (≤ 6 mice/cage) in sawdust-lined cages. All animal procedures were approved by the Institutional Ethics Committee.

### EXAMPLE 2

### Induction of mBSA/IL-1-induced arthritis (acute arthritis)

The procedure was based on that previously described (Lawlor et al., Arthritis and Rheumatism 44: 442-450, 2001). Mice were anaesthetized and injected intra-articularly. into the knee joint with 10 µl of 20 mg/ml mBSA (Sigma, St Louis, MO). Control joints received the same volume of vehicle (normal saline). Mice were next injected subcutaneously (s.c.) into the rear footpad with 20 µl of 12.5 µg/ml recombinant human IL-1β (Specific Activity 5 x 10⁸ U/mg; Amgen, Thousand Oaks, CA) in normal saline/0.5% (v/v) normal mouse serum (vehicle) and the injection was repeated on the next 2 days.

Mice were sacrificed on day 7 (or at indicated time points), the knee joints excised and fixed in 10% (v/v) neutral-buffered formalin for at least 2 days, decalcified and processed to paraffin. Frontal tissue sections (4 µm) were cut at 4 depths approximately 100 µm apart and stained with haematoxylin and eosin (H&E) to assess joint pathology.

Assessment of arthritis was performed blinded to the experimental groups. Five components of arthritis were assessed, i.e. joint space exudate, synovitis, pannus formation, cartilage and bone degradation. These were graded for severity from 0 (normal) to 5 (severe). Based on the histological scores, joints were classified as demonstrating inflammatory arthritis if there was an exudate score of 1 or more and synovitis score of 2 or more. Destructive arthritis was classified as a score of 2 or greater for pannus and 1 or greater for cartilage and/or bone degradation. The overall mean histological severity score was also calculated, with a maximum possible score per joint of 25 (Lawlor *et al.,* 2001, *supra).* Safranin O stained sections were prepared and assessed blindly for cartilage proteoglycan loss.

### EXAMPLE 3

### Induction of collagen induced arthritis (CIA)

Chick type II collagen (CII; Sigma) dissolved in 10 mM acetic acid overnight at 4°C at a concentration of 2 mg/ml, was emulsified in an equal volume of Freund's complete adjuvant (CFA), prepared at 5 mg/ml by adding heat-killed *Mycobacterium tuberculosis* (strain H37 Ra; Difco Laboratories, Detroit, MI, USA) to Freund's incomplete adjuvant (Difco). Mice were injected intra-dermally (i.d.) at several sites into the base of the tail with 100 µl of the emulsion and this was repeated 21 days later.

Animals were monitored for erythema and swelling of limbs and a clinical score given to each mouse 3 times a week for up to 40 days. The scoring system was as previously described (Campbell et al., European Journal of Immunology 30: 1568-1575), where 0 = normal, 1 = slight swelling, 2 = extensive swelling and 3 = joint distortion and/or rigidity and the maximum score per mouse was 12. Clinical assessments were completed by two independent investigators blinded to the experimental groups. At sacrifice, paws were removed, fixed, decalcified and processed for paraffin embedding as described above. H&E stained sections (5 µm) of the front and rear paws of four mice with the highest clinical scores were evaluated as previously described (Campbell *et al.,* 2000, *supra*). At day 30 and the day of sacrifice (day 62), blood was taken for determination of serum anti-CII Ab.

### EXAMPLE 4

### Administration of G-CSF

### Intra-articular G-CSF and IL-1

Mice received daily i.a. injection of 10 µl of IL-1 (25 ng) or recombinant human G-CSF (rHuG-CSF; 0.1, 0.5, 1 and 1.5 µg) or vehicle (saline; normal saline/0.5% (v/v) normal mouse serum (vehicle) on days 0, 1 and 2. Mice were sacrificed on day 3 and joints assessed histologically on H&E stained sections.

### Subcutaneous G-CSF in lieu of IL-1β in acute arthritis

Mice were injected i.a. with mBSA and treated s.c. in the footpad on days 0-2 with either IL-1 (250 ng) or rHuG-CSF [filgrastim] (15 µg) or vehicle control. Mice were sacrificed at day 7 as described above.

### Depletion of neutrophils in WT and G-CSF^{-/}⁻ mice

WT (B6) and G-CSF^{-/-} mice were treated intra-peritoneally 2 days prior to disease induction and on days 0 to 2 with 0.6 mg neutrophil-depleting monoclonal antibody (mAb), RB6.8C5 or isotype control mAb GL121. Mice were then treated daily from days 3 to 6 with 0.5 mg of mAb. Peripheral blood was analyzed for neutrophil counts on days 0, 2 and 7 by differential cell count analysis.

### EXAMPLE 6

### T cell proliferation assay

Inguinal lymph nodes (LN) were harvested from mice (n > 5 mice/experiment) immunized for CIA, 52-62 days after primary injection. Single cell suspensions were prepared in RPMI containing 2-mercaptoethanol (50 µM) and 5% (vol/vol) fetal bovine serum (FCS). LN cells (2 x 10⁵ cells) in 200 µl were plated in a round-bottomed 96-well plate (Becton Dickinson Labware, Franklin Lakes, New Jersey, USA) and stimulated with 0-100 µg/ml denatured CII (boiled 10 minutes). Cells were incubated for 72 hours at 37°C (5% CO₂), supernatants taken at 20 and 48 hours, and pulsed for the final 8h with 1 µCᵢ [³H] thymidine. Cells were harvested with an Inotech Cell Harvester (Inotech) and [³H] thymidine incorporation was measured as a measure of T cell proliferation using a microplate scintillation counter (Canberra Packard, Victoria, Australia). Aliquots of cell supernatants were taken at 20 and 48 hours.

### Flow cytometric analysis of infiltrating leukocytes

Patellae and attached soft tissues from mBSA/saline-treated B6 mice and mBSA/IL-1-treated B6 and *G-CSF-*/*-* mice were dissected on days 3 and 7. Bone, fat and muscle fragments were discarded, the synovium was minced into 2-mm pieces and digested into a single cell suspension using 2.4 mg/ml dispase II (Boehringer, Mannheim, Germany), 1 mg/ml collagenase type II (Sigma), and 100 µg/ml DNAse I (Boehringer Mannheim, Indianapolis, USA) in RPMI 1640. The suspension was gently agitated at 37 °C for 45 min and then washed through a 70 µM nylon cell strainer (Falcon) with 10% [v/v] FCS in RPMI. Cell counts were performed prior to cell staining. Non-specific-staining was blocked using rat anti-mouse FcγRIIb/III (CD16/CD32; clone 2.4G2; American Type Culture Collection (ATCC), Manassas, Virginia, USA). Leukocytes were stained using biotinylated rat anti-mouse CD45.2 (PharMingen, San Diego, California, USA) and fluorochrome streptavidin tricolor (SA-TRI) (Caltag) and combinations of Ab listed below in EXAMPLE 12. Synovial digest cells were also adhered overnight at 37 °C, 5% CO₂ to recover expression of markers such as CD4 that are cleaved by dispase. Non-adherent cells were collected in 2% v/v FCS in PBS and stained for fluorescence activated cell sorting (FACS).

### EXAMPLE 7

### Cytokine assays and leukocyte morphological quantification

IFN-γ, IL-4 and IL-2 were measured in T cell supernatants by capture ELISAs using paired monoclonal antibodies, according to the manufacturer's instructions (Pharmingen).

### Joint exudate leukocyte morphological quantification

H&E stained joint sections (n = 2 section depths per joint) were analysed for exudate composition by quantifying polymorphonuclear leukocytes, monocyte/macrophages and lymphocytes in 5 grid areas of focal joint space exudate at high magnification (1000x).

### EXAMPLE 8

### Determination of serum anti-CII antibodies (Ab)

ELISAs were performed to detect Abs to CII as previously described (Campbell *et al.,* 2000, *supra).* Horseradish peroxidase-conjugated goat anti-mouse IgG (Sigma Chemical Co.), IgG2b, IgG2c, IgG1, IgG3 or IgM (Southern Biotechnology Associates, Birmingham, Alabama, USA) antisera were used as detection Abs. Standard curves were constructed from pooled sera of hyper-immunized DBA/1 mice using arbitrary units.

### Determination of serum Ig levels

Serum Abs from retro-orbital sinus bleeds of naïve and CII/CFA-immunised mice were captured with relevant Abs to total IgG, IgM, IgG2c, IgG2b, IgG1, IgG3 and IgA and detected using horseradish peroxidase (HRP) conjugated Abs (Southern Biotechnology Associates, Birmingham, Alabama, USA).

### EXAMPLE 9

### Peripheral blood leukocyte counts

Peripheral blood was obtained from mice by retro-orbital plexus venesection on days 0, 3 and 7 of the acute arthritis model, and collected in EDTA coated tubes. BM was flushed from one femur per mouse into 3% [v/v] foetal calf serum (FCS)/ phosphate buffered saline (PBS). Total leukocyte counts and differential analyses were performed using an Advia 120 Hematology System (Bayer Diagnostics, Tarrytown, New York, USA). Manual BM counts were also done on cytospins (1x10⁵ cells centrifuged onto a microscope slide at 1200 rpm for 7 min).

### EXAMPLE 10

### Joint inflammation develops in response to direct intra-articular administration of G-CSF

It was first investigated whether granulocyte-colony stimulating factor (G-CSF) has pro-inflammatory properties in joints by intra-articular injection of rHuG-CSF (0.1, 0.5, & 1 µg) into the knee joint of wild type (WT) C57BL/6 mice over three consecutive days. Controls included intra-articular injection of IL-1 (IL-1; 25ng) and vehicle (0.5% [v/v] normal mouse serum in normal saline). On day 3 joints were taken for histological assessment. It was found that G-CSF induced inflammation in a dose-dependent manner (Figure 1), although the response was significantly less than that induced with IL-1. This result shows that exogenous G-CSF has pro-inflammatory effects within the normal joint.

### EXAMPLE 11

### G-CSF-deficient mice develop less IL-1 induced joint inflammation

To investigate the involvement of G-CSF in modulating local IL-1-induced inflammatory effects, mice deficient in G-CSF *(G-CSF-*/*-)* and B6 mice were injected i.a. with IL-1. IL-1 induced joint inflammation in *G-CSF-*/*-* mice, but at a reduced level compared to normal B6 mice (Figure 2A), demonstrating that G-CSF is a downstream mediator of IL-1 in the joint compartment. Despite this reduction in inflammatory features in *G-CSF-*/*-* mice, there was no difference in articular cartilage proteoglycan loss (Figure 2B), suggesting that IL-1 can directly damage cartilage.

### Peripheral blood, BM and synovial infiltrate analysis

To examine the effects of G-CSF deficiency on the inflammatory response induced by mBSA and IL-1, peripheral blood, BM and synovium were taken from B6 and *G-CSF-*/*-* mice treated i.a. with mBSA and s.c. with either IL-1 or saline vehicle, at days 0, 3 and 7 of this model. Results are shown in Table 2 and 3, and in Figure 6. G-CSF deficiency resulted in a blunted neutrophilic response to mBSA/IL-1. BM cellularity in both B6 and *G-CSF-*/*-* mice was reduced by IL-1 administration (Table 2), reflecting the mobilisation of BM cells into the blood. When compared with B6 mice, *G-CSF-*/*-* mice had significantly reduced numbers of metamyelocytes and polymorphs, as well as fewer promyelocytes and myelocytes in the BM compartment (Table 2) both basally and in response to IL-1. B6 mice developed a marked peripheral neutrophilia during the course of acute arthritis. In sharp contrast, *G-CSF-*/*-* mice exhibited a significant neutropenia over the course of the model (Table 3), indicating that the neutrophilia induced by IL-1 is G-CSF dependent.

Investigation of the cellular composition of the inflamed synovium of mBSA/IL-1-treated G-CSF-/- mice also revealed a reduction in infiltrating leukocytes at day 3 and 7 (Figure 6A-C). Cell counts of synovial tissue digests revealed approximately 2-fold reduction in total cellularity at day 7 in mBSA/IL-1-treated G-CSF-/- joint synovial tissue, compared to mBSA/IL-1-treated synovial tissue from B6 mice (1.87 ± 0.07 x 10⁵ cells/ B6 mBSA/saline joint versus 3.13 ± 0.10 x 10⁵ cells/ B6 mBSA/IL-1 joint versus 1.66 ± 0.05 x 10⁵ cells/ G-CSF-/- mBSA/IL-1 joint). In particular, there were significant reductions in the percentages and numbers of neutrophils (GR1hi CD11bhi) at both time points, as well as reductions in the percentage and number of infiltrating macrophage/monocytes (GR11o CD11bhi). Other phenotypic changes included lower percentages of M-CSFR⁺ CD16/CD32⁺ and CD44⁺ cells in the synovial tissues of G-CSF-/- mice with acute arthritis, suggesting that G-CSF may be required for the full induction of these activation markers on synovial cells.

Staining of non-adherent leukocytes after overnight culture also revealed a reduction in CD4⁺ lymphocyte infiltration at day 7, suggesting that G-CSF is required for trafficking of not only neutrophils and macrophages into the joint but also of CD4⁺ T lymphocytes (Figure 6C).

### EXAMPLE 12

### Depletion of neutrophils in G-CSP^{-/}⁻ and WT mice in acute inflammatory arthritis

To investigate whether the reduction in mBSA/IL-1-induced arthritis in G-CSF^{-/-} mice was simply a result of neutropenia (Lieschke *et al.,* 1994, *supra*)*,* neutrophils were depleted using the monoclonal antibody (mAb), RB6.8C5. WT and G-CSF^{-/-} mice were injected intra-peritoneally (i.p.) with anti-neutrophil mAb (RB6.8C5) or isotype control mAb (GL121). Peripheral blood was analyzed for neutrophil levels on days 0, 2 and 7 by differential cell count analysis (Figure 7A). In WT animals treated with anti-neutrophil mAb, >90% depletion was observed at all times compared to isotype control mAb treated animals (which developed a marked neutrophilia). Neutrophil depletion of WT mice did not abrogate development of arthritis (Figure 7B), although it did significantly decrease the joint space exudate. In contrast, G-CSF^{-/-} mice were relatively resistant to disease and additional neutrophil depletion did not further reduce disease severity. This indicates that the reduction in neutrophils in the G-CSF^{-/-} mice is not solely responsible for protection from mBSA/IL-1-induced arthritis.

### EXAMPLE 13

### Exogenous G-CSF can partly substitute for IL-1 in acute inflammatory arthritis

Joints of mice treated with mBSA and G-CSF (mBSA/G-CSF) developed inflammatory and destructive arthritis, although this was less severe than mBSA/IL-1-treated animals (Figure 4A-B). The major cells infiltrating joints of the mBSA/G-CSF-treated animals were monocyte/macrophages, compared to the predominantly granulocytic infiltrate in mBSA/IL-1-induced arthritis. These results show that systemic administration of exogenous G-CSF can at least partially substitute for systemic IL-1 in driving this model of acute arthritis and that G-CSF leads to the recruitment of monocyte/macrophages into the joint.

### EXAMPLE 14

### G-CSF deficiency impairs collagen-induced arthritis (CIA)

### G-CSF-deficient mice have reduced acute inflammatory arthritis

In view of the pro-inflammatory effects of i.a. injection and systemic *filgrastim* on joint disease, we attempted to determine the absolute dependence of the acute arthritis model on G-CSF, using *G-CSF-*/*-* mice. *G-CSF-*/*-* and B6 mice were injected i.a. with mBSA (day 0) and s.c. in the footpad with IL-1 on days 0, 1 and 2. Histological assessment of disease at day 7 revealed a significant reduction in inflammatory and destructive features (Figure 5A & B). Safranin O staining for cartilage proteoglycan content revealed a major reduction in cartilage proteoglycan loss in *G-CSF-*/*-* mice compared to B6 mice (Figure 5C). Therefore, endogenous G-CSF is an important mediator of inflammation and destruction in this model of acute arthritis.

### G-CSF deficiency reduces the incidence and severity of CIA

CIA is a chronic autoimmune arthritis that is widely used to study RNA. To examine the contribution of G-CSF to CIA, B6 WT and *G-CSF-*/*-* mice were immunised with CII in CFA, followed by a boost injection 21 days later (Lieschke et al., Blood 84:1737-46, 1994), and disease incidence and severity compared. The onset of CIA in *G-CSF-*/*-* mice was delayed and mice developed disease at a markedly reduced incidence and severity compared to WT mice (Figure 8A & B). The reduction of disease incidence and severity in *G-CSF-*/*-* mice suggests a pivotal role for endogenous G-CSF in chronic autoimmune arthritis.

### EXAMPLE 15

### Histological analysis of CIA in G-CSF^{-/}⁻ mice

Histological assessment of H&E stained sections of paws from four B6 and *G-CSF-l-* mice with the highest clinical scores during CIA was performed. Individual joints were scored from 0 normal to 3 (see EXAMPLE 18) and the percentage of normal and arthritic joints determined. There was a significantly greater percentage of normal joints in *G-CSF-*/*-* mice compared to WT mice (Figure 9A), and of the small number of affected paws in the G-*CSF-*/*-* mice, none was severe (Figure 9B). In contrast, joints from WT mice had a range of histological features indicative of mild to severe arthritis (Figure 9A & B). These histological observations are concordant with the clinical assessment outlined herein.

### EXAMPLE 16

### In vitro T cell proliferation & cytokine production to CII

To assess the cellular immune response to CII, in vitro T cell proliferative responses and T cell cytokine (IFNγ and IL-2) production were measured in *G-CSF-*/*-* mice and compared to WT. Single cell suspensions were prepared from inguinal LN from mice immunised with CII in CFA and stimulated for 72h *in vitro* with 0 - 100 µg/ml of denatured CII. T cell responses were measured by tritiated TdR uptake in the last 8 h of culture. Figure 10A depicts the stimulation indices observed in *G-CSF-*/*-* and WT mice, which were comparable. Production of T cell cytokines IFN-γ and IL-2 by *G-CSF-*/*-* LN cells appeared relatively normal (Figure 10B).

### EXAMPLE 17

### Impaired anti-CII isotype switching from IgM to IgG in G-CSF-/- mice

Induction of CIA is dependent on both humoral and cellular immune responses to CII (Campbell *et al.,* 2000, *supra*)*.* It was examined whether G-CSF deficiency altered serum levels of anti-CII Ab production during CIA (day 30 and 62). Despite comparable levels of anti-CII IgM in WT and *G-CSF-*/*-* mice at days 30 and 62, there was a reduction in the level of total anti-CII IgG (Figure 11). Analysis of anti-CII IgG isotypes revealed reduced production of all isotypes - IgG2b, IgG2c, IgG3 and IgG1. This suggests that there is a defect in isotype switching in *G-CSF-*/*-* mice that may contribute to protection against CIA. This observation suggests that endogenous G-CSF plays a role in Ab production by B cells, at least in response to immunisation with antigen in CFA.

### EXAMPLE 18

### Increased basal levels of Ig in naive G-CSF-/- mice and increased total IgG in CII/CFA-immunised mice

Investigation of the humoral immune response to CII revealed that *G-CSF-*/*-* mice have defective switching from an IgM to IgG response. To determine whether this defect reflected pre-existing defects in circulating Ab, sera from naïve mice were analysed for total IgG, IgM and IgG isotypes. Analysis of serum Ab levels revealed significantly greater production of total IgG and isotypes IgG2b and IgG2c in naive *G-CSF-*/*-* mice compared to naïve B6 mice and normal baseline production of IgM, IgG1, IgG3 and IgA (Figure 12A). Furthermore, in CII/CFA-immunised *G-CSF-*/*-* mice, levels of non-specific total IgG were markedly enhanced (Figure 12B). These observations suggest G-CSF plays a role in B cell maturation, antibody production and isotype switching, at least in response to immunisation with antigen in CFA.

### EXAMPLE 19

### Normal T-dependent and T-independent Ag B cell responses in G-CSF-/- mice

To determine whether the impairment of Ab production in response to CII/CFA was due to a defect in T-dependent or T-independent Ag responses, B6 and *G-CSF-*/*-* mice were challenged with the T-dependent antigen NP-KLH in alum, or with the T-independent antigen, DNP-Dextran in PBS. *G-CSF-*/*-* mice developed a normal response to both T-dependent and T-independent Ag (Figure 13), demonstrating that the impaired B cell response in CIA is specific to challenge with CII in CFA.

### EXAMPLE 20

### G-CSF depletion causes a Peripheral blood neutropenia and reduces inflammation in acute (mBSA/IL-1) arthritis

To assess the therapeutic application of G-CSF blockade in WT mice, B6 mice were injected prior to the induction of acute arthritis (day 0) with 50 or 250 µg of rat anti-G-CSF mAb (clone 67604; R&D systems, Minneapolis, Minnesota, USA) or isotype control (GL113) mAb. mAb was also administered on days 1, 2, 3 and 5. Peripheral blood was taken at days 4 and 7 and subjected to differential analysis (Figure 14A). Mice receiving the high dose (250 µg) anti-G-CSF mAb had a significant reduction in peripheral blood neutrophils compared to isotype control mAb mBSA/IL-1-treated mice on both days. Mice receiving the lower dose of anti-G-CSF mAb had a significant reduction in neutrophils at day 7 only. Analysis of BM populations also showed a significant reduction in myeloid lineage cells in G-CSF-depleted mBSA/IL-1-treated mice (data not shown). Macroscopic assessment of arthritic joints of mice treated with anti-G-CSF or isotype control mAb revealed a reduction in disease in anti-G-CSF mAb (250 µg) treated mice (Figure 14B). There was reduced synovial tissue cellularity (Figure 14C), and a reduced percentage of infiltrating leukocytes, especially neutrophils (GR1*hi* CD11b*hi*; Figure 14D), in mice receiving the higher dose of anti-G-CSF mAb. These results show a dose-dependent reduction in disease features of acute arthritis in response to G-CSF inhibition in wild type mice.

**TABLE 2**

| ***Bone marrow populations during mBSA*/*IL-1-induced arthritis*** | | | | | | | | | |
|---|---|---|---|---|---|---|---|---|---|
| Cells x 10⁴ /femur | | | | | | | | | |
| Genotype | Study day | Total cellularity | Blast | Promyelocyte/M yelocyte | Metamyelocyte /Polymorph | Lymphocyte | Monocyte | Eosinophil | Nucleated erythroid cell |
| B6 | 0(2) | 2134 ± 103 | 72 ± 2 | 102 ± 4 | 777 ± 4 | 409 ± 18 | 92 ± 2 | 92 ± 7 | 501 ± 2 |
| G-CSF^{-/-} | 0(2) | 1902 ± 402† | 16 ± 0† | 58 ± 0 | 142 + 2† | 342 ± 2 | 100 ± 1 | 58 ± 1 | 336 ± 1 † |
| B6 | 3 (5) | 1456 ± 79‡ | 23 ± 4‡ | 150 ± 6 | 601 ± 38 | 173 ± 27 | 152 ± 7 | 40 ± 2 ‡ | 299 ± 24 |
| G-CSF^{-/-} | 3 (5) | 1216 ± 97‡ | 41 ± 6 | 80 ± 7† | 229 ± 21 † | 263 ± 18 | 110 ± 13 | 32 ± 4 | 324 ± 28 |
| B6 | 7 (6) | 1710 ± 227 | 21 ± 4‡ | 49 ± 4‡ | 750 ± 14 | 92 ± 12‡ | 99 ± 7 | 54 ± 2 | 213 ± 10 ‡ |
| G-CSF^{-/-} | 7(6) | 1458 ± 147 | 34 ± 2‡ | 34 ± 2 †‡ | 343 ± 36 †‡ | 279 ± 18 | 121 ± 10 | 23 ± 6 † | 460 ± 44 † |

| | | | | | | | | | |
|---|---|---|---|---|---|---|---|---|---|
| Cell counts were performed using the Advia counter. Manual leukocyte differentials were performed on diff-quik stained cytospins on cells recovered from one femur per mouse. Values are the mean ± SD. * Study day 0 is pretreatment; the value in parentheses is the number of mice studied for that group. † P< 0.05 for comparison between B6 and G-CSF^{-/-} mice on the same study day ‡ P< 0.05 between mice of the same genotype at baseline and on the study day indicated | | | | | | | | | |

**TABLE 3**

| ***Peripheral blood analysis of G-CSF*^{*-*/}*⁻ mice during mBS4*/*IL-1-induced arthritis*** | | | | | |
|---|---|---|---|---|---|
| Genotype | Day of model* | Cells x 10³ per µl of Peripheral blood | | | |
| | | Total WBC | Lymphocytes | Neutrophils | Monocytes |
| B6 | 0 (5) | 3.77 ± 1.00 | 3.17 ± 0.83 | 0.33 ± 0.06 | 0.04 ± 0.02 |
| G-CSF^{-/-} | 0(4) | 7.51 ± 0.49† | 6.88 ± 0.38† | 0.19 ± 0.03 | 0.03 ± 0.01 |
| B6 | 3 (9) | 2.55 ± 0.30 | 1.89 ± 0.26 | 0.40 ± 0.05 | 0.02 ± 0.00 |
| G-CSF^{-/-} | 3 (9) | 3.15 ± 0.24 | 2.75 ± 0.18† | 0.12 ± 0.03† | 0.03 ± 0.01 |
| B6 | 7 (9) | 3.68 ± 0.80 | 2.68 ± 0.66 | 0.63 ± 0.11 | 0.06 ± 0.02 |
| G-CSF^{-/-} | 7(9) | 3.28 ± 0.38 | 2.93 ± 0.33 | 0.20 ± 0.04† | 0.03 ± 0.00 |

| | | | | | |
|---|---|---|---|---|---|
| Peripheral blood was analysed using the Advia counter and cell counts x 10³ per µl determined. Data are represented as mean ± SEM. * Study day 0 is pre-treatment baseline counts; number in parentheses is the number of mice examined for that group. † P < 0.05 between groups on time point examined. | | | | | |

### BIBLIOGRAPY

Bungart et al., British Journal of Haematology 22: 1156, 1990;
Campbell et al., Journal of Leukocyte Biology 68: 144-150, 2000;
Campbell et al., European Journal of Immunology 30: 1568-1575, 2000;
Colotta et al., Blood 80: 2012-2020, 1992;
Demetri and Griffin, Blood 78: 2791-2808,1991;
Douillard and Hoffman, Basic Facts about Hybridomas, in Compendium of Immunology Vol. II, ed. by Schwartz, 1981;
Foster et al., Transplantation 59: 1557, 1995;
Franzke et al., Blood 102: 734-39, 2003;
Gericke et al., Journal ofLeukocyte Biology 57: 455-461, 1995;
Gorgen et al., Journal of Immunology 149: 918, 1992;
de Haan et al., Blood. 86: 2986-2992, 1995;
Hamilton et al., Blood 79: 1413-1419, 1992;
Jacob et al., Blood 92: 353-361, 1998;
Kitching et al., Journal of the American Society of Nephrology 13: 350-358, 2002;
Kohler and Milstein, Nature 256: 495-499, 1975;
Kohler and Milstein, European Journal of Immunology 6: 511-519, 1976;
Lawlor et al., Arthritis and Rheumatism 44: 442-450, 2001;
Leizer et al., Blood 76: 1989-1996, 1990;
Lieschke et al., Blood 84: 1737-1746, 1994;
Lord et al., Proc. Natl. Acad. Sci. USA 86: 9499-9503, 1989;
Metcalf, International Journal of Cancer, 25: 225, 1980;
Miyahara et al., Clinical Immunology and Immunopathology 69: 69-76, 1993;
Nakamura et al., Clinical and Experimental Rheumatology 18: 713-718, 2000;
Nicola et al., Nature 314: 625, 1985;
Nicola et al., Journal of Biological Chemistry 258: 9017, 1983;
Pan et al., Blood 86: 4422, 1995;
Rex et al., Transfusion 35: 605-611, 1995;
Roberts et al., Blood 89: 2736-2744, 1997;
Sambrook et al., Molecular Cloning - A Laboratory Manual, Cold Spring Harbour, New York, USA, 1990;
Snowden et al., Bone Marrow Transplantation 22: 1035-1041, 1998;
Souza et al., Science 232: 61, 1986;
Takahashi et al., Laboratory Investigation 74: 827-834, 1996;
Tanabe et al., Rheumatology International 16: 67-76, 1996;
Welte et al., Blood 88: 1907-1929, 1996;
Xu et al., British Journal of Haematology 93: 558-568, 1996;
Yong and Linch, European Journal of Haematology 49: 251-259, 1992;
Yong, British Journal of Haematology 94: 40-47, 1996;
Zavala et al., Journal of Immunology 163: 5125-5132, 1999;
Zavala et al., Journal of Immunology 168: 2011-2019, 2002;

## Claims

1. An agent which inhibits the activity of granulocyte-colony stimulating factor (G-CSF), or granulocyte-colony stimulating factor receptor (G-CSFR) and/or which reduces the level of expression of a gene encoding said G-CSF or G-CSFR for treatment or prophylaxis of arthritis in a subject.

2. The agent of Claim 1, wherein the arthritis is chronic inflammatory arthritis or rheumatoid arthritis (RA) or collagen induced arthritis (CIA).

3. The agent of any one of Claims 1 or 2, wherein the subject is an animal or avian species.

4. The agent of Claim 3, wherein the animal is a mammal.

5. The agent of Claim 4, wherein the mammal is a primate or rodent.

6. The agent of Claim 5, wherein the primate is a human or the rodent is a mouse.

7. The agent of any one of Claims 1 to 6, wherein the agent is an antibody to G-CSF or G-CSFR or an epitope thereon.

8. The agent of Claim 7, wherein the antibody is a monoclonal antibody or a polyclonal antibody.

9. The agent of any one of Claims 1 to 6, wherein the agent is a soluble form of G-CSFR.

10. The agent of any one of Claims 1 to 6, wherein the agent is a chemical analog of G-CSF or a chemical analog of G-CSFR, and wherein said chemical analogs are antagonists of G-CSF or G-CSFR.

11. The agent of any one of Claims 9 or 10, wherein the agent is a protein.

12. The agent of any one of Claims 1 to 6, wherein the agent is a nucleic acid.

13. The agent of Claim 12, wherein the nucleic acid is DNA or RNA and comprises a sense or antisense polynucleotide sequence or a genetic sequence encoding G-CSF or G-CSFR.

14. Use of an agent which inhibits the activity of granulocyte-colony stimulating factor (G-CSF), or granulocyte-colony stimulating factor receptor (G-CSFR) and/or which reduces the level of expression of a gene encoding said G-CSF or G-CSFR, in the manufacture of a medicament for the treatment or prophylaxis of arthritis in a subject, wherein said arthritis, subject and agent are as defined in one or more of Claims 1 to 13.

15. An in-vivo non-therapeutic method for identifying agents capable of inhibiting the activity of G-CSF and/or inhibiting the interaction of G-CSF with G-CSFR, said method comprising administering a putative inhibitory agent to a genetically modified non-human animal which has been modified to produce low amounts of G-CSF or G-CSFR relative to a non-genetically modified animal of the same species, wherein said agent is identified as having interactivity with G-CSF or G-CSFR by the agent having a detectable physiological effect in a wild type animal of the same species, but a reduced effect in an animal which exhibits reduced expression of G-CSF and/or G-CSFR.

## Patentansprüche

1. Mittel, das die Aktivität von Granulocyten-koloniestimulierendem Faktor (G-CSF) oder Granulocyten-koloniestimulierender-Faktor-Rezeptor (G-CSFR) hemmt und/oder das Expressionsniveau eines Gens, das den G-CSF oder G-CSFR codiert, reduziert, zur Behandlung oder Prophylaxe von Arthritis bei einem Patienten.

2. Mittel gemäß Anspruch 1, wobei die Arthritis eine chronische entzündliche Arthritis oder rheumatoide Arthritis (RA) oder collageninduzierte Arthritis (CIA) ist.

3. Mittel gemäß einem der Ansprüche 1 oder 2, wobei der Patient eine Tier- oder Vogelart ist.

4. Mittel gemäß Anspruch 3, wobei das Tier ein Säuger ist.

5. Mittel gemäß Anspruch 4, wobei der Säuger ein Primat oder Nager ist.

6. Mittel gemäß Anspruch 5, wobei der Primat ein Mensch oder der Nager eine Maus ist.

7. Mittel gemäß einem der Ansprüche 1 bis 6, wobei das Mittel ein Antikörper gegen G-CSF oder G-CSFR oder ein darauf liegendes Epitop ist.

8. Mittel gemäß Anspruch 7, wobei der Antikörper ein monoklonaler Antikörper oder ein polyklonaler Antikörper ist.

9. Mittel gemäß einem der Ansprüche 1 bis 6, wobei das Mittel eine lösliche Form von G-CSFR ist.

10. Mittel gemäß einem der Ansprüche 1 bis 6, wobei das Mittel ein chemisches Analogon von G-CSF oder ein chemisches Analogon von G-CSFR ist und wobei die chemischen Analoga Antagonisten von G-CSF oder G-CSFR sind.

11. Mittel gemäß einem der Ansprüche 9 oder 10, wobei das Mittel ein Protein ist.

12. Mittel gemäß einem der Ansprüche 1 bis 6, wobei das Mittel eine Nucleinsäure ist.

13. Mittel gemäß Anspruch 12, wobei die Nucleinsäure eine DNA oder RNA ist und eine Sense- oder Antisense-Polynucleotidsequenz oder eine genetische Sequenz, die G-CSF oder G-CSFR codiert, umfasst.

14. Verwendung eines Mittels, das die Aktivität von Granulocyten-koloniestimulierendem Faktor (G-CSF) oder Granulocyten-koloniestimulierender-Faktor-Rezeptor (G-CSFR) hemmt und/oder das Expressionsniveau eines Gens, das den G-CSF oder G-CSFR codiert, reduziert, bei der Herstellung eines Medikaments zur Behandlung oder Prophylaxe von Arthritis bei einem Patienten, wobei die Arthritis, der Patient und das Mittel wie in einem oder mehreren der Ansprüche 1 bis 13 definiert sind.

15. Nichttherapeutisches in-vivo-Verfahren zum Identifizieren von Mitteln, die die Aktivität von G-CSF hemmen und/oder die Wechselwirkung von G-CSF mit G-CSFR hemmen kann, wobei das Verfahren das Verabreichen eines mutmaßlichen Inhibitors an ein genetisch modifiziertes nichthumanes Tier umfasst, das so modifiziert wurde, dass es im Vergleich zu einem nicht genetisch modifizierten Tier derselben Spezies nur geringe Mengen an G-CSF oder G-CSFR produziert, wobei das Mittel anhand seiner Wechselwirkung mit G-CSF oder G-CSFR identifiziert wird, und zwar dadurch, dass das Mittel bei einem Wildtyptier derselben Spezies eine nachweisbare physiologische Wirkung hat, aber bei einem Tier, das eine reduzierte Expression von G-CSF und/oder G-CSFR aufweist, eine reduzierte Wirkung hat.

## Revendications

1. Agent qui inhibe l'activité du facteur stimulant les colonies de granulocytes (G-CSF), ou d'un récepteur du facteur stimulant les colonies de granulocytes (G-CSFR) et/ou qui réduit le degré d'expression d'un gène codant ledit G-CSF ou G-CSFR pour le traitement ou la prophylaxie d'une arthrite chez un sujet.

2. Agent selon la revendication 1, dans lequel l'arthrite est une arthrite inflammatoire chronique ou une polyarthrite rhumatoïde (RA) ou une arthrite induite par le collagène (CIA).

3. Agent selon l'une quelconque des revendications 1 ou 2, dans lequel le sujet est une espèce animale ou aviaire.

4. Agent selon la revendication 3, dans lequel l'animal est un mammifère.

5. Agent selon la revendication 4, dans lequel le mammifère est un primate ou un rongeur.

6. Agent selon la revendication 5, dans lequel le primate est un être humain ou le rongeur est une souris.

7. Agent selon l'une quelconque des revendications 1 à 6, l'agent étant un anticorps contre le G-CSF ou le G-CSFR ou un épitope sur ceux-ci.

8. Agent selon la revendication 7, dans lequel l'anticorps est un anticorps monoclonal ou un anticorps polyclonal.

9. Agent selon l'une quelconque des revendications 1 à 6, l'agent étant une forme soluble du G-CSFR.

10. Agent selon l'une quelconque des revendications 1 à 6, l'agent étant un analogue chimique du G-CSF ou un analogue chimique du G-CSFR, et dans lequel lesdits analogues chimiques sont des antagonistes du G-CSF ou du G-CSFR.

11. Agent selon l'une quelconque des revendications 9 ou 10, l'agent étant une protéine.

12. Agent selon l'une quelconque des revendications 1 à 6, l'agent étant un acide nucléique.

13. Agent selon la revendication 12, dans lequel l'acide nucléique est un ADN ou un ARN et comprend une séquence polynucléotidique sens ou antisens ou une séquence génétique codant le G-CSF ou le G-CSFR.

14. Utilisation d'un agent qui inhibe l'activité du facteur stimulant les colonies de granulocytes (G-CSF), ou d'un récepteur du facteur stimulant les colonies de granulocytes (G-CSFR) et/ou qui réduit le degré d'expression d'un gène codant ledit G-CSF ou G-CSFR, dans la fabrication d'un médicament pour le traitement ou la prophylaxie d'une arthrite chez un sujet, dans laquelle lesdits arthrite, sujet et agent sont tels que définis dans une ou plusieurs des revendications 1 à 13.

15. Procédé non thérapeutique in vivo pour identifier des agents aptes à inhiber l'activité du G-CSF et/ou à inhiber l'interaction du G-CSF avec le G-CSFR, ledit procédé comprenant l'administration d'un agent inhibiteur putatif à un animal non humain génétiquement modifié qui a été modifié pour produire de faibles quantités de G-CSF ou de G-CSFR par rapport à un animal non génétiquement modifié de la même espèce, dans lequel ledit agent est identifié comme ayant une interactivité avec le G-CSF ou le G-CSFR par l'agent ayant un effet physiologique détectable chez un animal de type sauvage de la même espèce, mais un effet réduit chez un animal qui présente une expression réduite du G-CSF et/ou du G-CSFR.
